# EUROPEAN PATENT SPECIFICATION

(11) **EP 2 303 336 B1**
(45) Date of publication and mention of the grant of the patent: **23.03.2016**
(21) Application number: 09784667.9
(22) Date of filing: 09.07.2009
(51) Int. Cl.: A61K 48/00, A61K 31/455, A61K 31/565, A61K 31/57, C12Q 1/68, A61P 9/10, A61P 19/00, A61P 21/00

(54) **SULFONYLUREA RECEPTOR AND MEANS FOR TREATING ISCHAEMIA**
SULFONYLHARNSTOFFREZEPTOR UND MITTEL ZUR BEHANDLUNG VON ISCHÄMIE
RÉCEPTEUR À UNE SULFONYLURÉE ET MOYENS POUR TRAITER L'ISCHÉMIE

(30) Priority: 11.07.2008 GB 0812659; 21.07.2008 GB 0813292
(43) Date of publication of application: 06.04.2011
(73) Proprietor: The University Of Dundee, Dundee 441 4HN (GB)
(72) Inventor: JOVANOVIC, Aleksandar, Dundee DD1 9SY (GB)
(74) Representative: D'Arcy, Julia
(86) International application number: PCT/GB2009/001705
(87) International publication number: WO 2010/004283

(56) References cited:
- DU QINGYOU ET AL: "Overexpression of SUR2A generates a cardiac phenotype resistant to ischemia" FASEB JOURNAL, vol. 20, no. 8, June 2006 (2006-06), pages 1131-1141, XP002556068 ISSN: 0892-6638
- DATABASE BIOSIS [Online] BIOSCIENCES INFORMATION SERVICE, PHILADELPHIA, PA, US; November 2001 (2001-11), CHEN CHAO-FUH ET AL: "The protective effect of niacinamide on ischemia-reperfusion-induced liver injury" XP002556070 Database accession no. PREV200200076834 & JOURNAL OF BIOMEDICAL SCIENCE, vol. 8, no. 6, November 2001 (2001-11), pages 446-452, ISSN: 1021-7770
- DATABASE BIOSIS [Online] BIOSCIENCES INFORMATION SERVICE, PHILADELPHIA, PA, US; March 2006 (2006-03), FENG YANGZHENG ET AL: "Nicotinamide reduces hypoxic ischemic brain injury in the newborn rat" XP002556071 Database accession no. PREV200600341276 & BRAIN RESEARCH BULLETIN, vol. 69, no. 2, March 2006 (2006-03), pages 117-122, ISSN: 0361-9230
- DATABASE BIOSIS [Online] BIOSCIENCES INFORMATION SERVICE, PHILADELPHIA, PA, US; May 2003 (2003-05), CAVASIN MARIA A ET AL: "Estrogen and testosterone have opposing effects on chronic cardiac remodeling and function in mice with myocardial infarction." XP002556072 Database accession no. PREV200300330090 & AMERICAN JOURNAL OF PHYSIOLOGY, vol. 284, no. 5 Part 2, May 2003 (2003-05), pages H1560-H1569, ISSN: 0002-9513
- DATABASE WPI Week 198501 Thomson Scientific, London, GB; AN 1985-005558 XP002556075 & SU 1 097 334 A (LATV CARDIOLOGY RES) 15 June 1984 (1984-06-15)
- BROWN DAVID A ET AL: "Susceptibility of the heart to ischaemia-reperfusion injury and exercise-induced card ioprotection are sex-dependent in the rat" JOURNAL OF PHYSIOLOGY (OXFORD), vol. 564, no. 2, April 2005 (2005-04), pages 619-630, XP002556069 ISSN: 0022-3751
- DATABASE BIOSIS [Online] BIOSCIENCES INFORMATION SERVICE, PHILADELPHIA, PA, US; March 2005 (2005-03), JOHNSON MICAH S ET AL: "Short-term exercise decreases infaret size in a sex-dependent manner and correlates with increased mycocardial KATP channel protein" XP002556073 Database accession no. PREV200510319860 & FASEB JOURNAL, vol. 19, no. 5, Suppl. S, Part 2, March 2005 (2005-03), page A1323, EXPERIMENTAL BIOLOGY 2005 MEETING/35TH INTERNATIONAL CONGRESS OF PHYSIOLOGICAL SCIENCES; SAN DIEGO, CA, USA; MARCH 31 -APRIL 06, 2005 ISSN: 0892-6638
- DATABASE BIOSIS [Online] BIOSCIENCES INFORMATION SERVICE, PHILADELPHIA, PA, US; September 2001 (2001-09), RANKI HARRI J ET AL: "Gender-specific difference in cardiac ATP-sensitive K+ channels" XP002556074 Database accession no. PREV200100480821 & JOURNAL OF THE AMERICAN COLLEGE OF CARDIOLOGY, vol. 38, no. 3, September 2001 (2001-09), pages 906-915, ISSN: 0735-1097

## Description

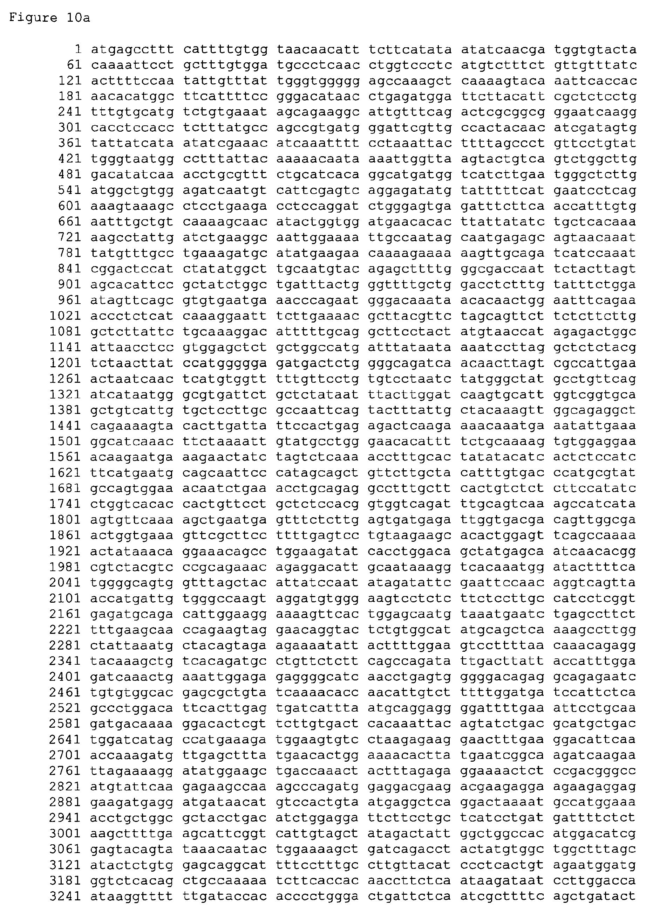

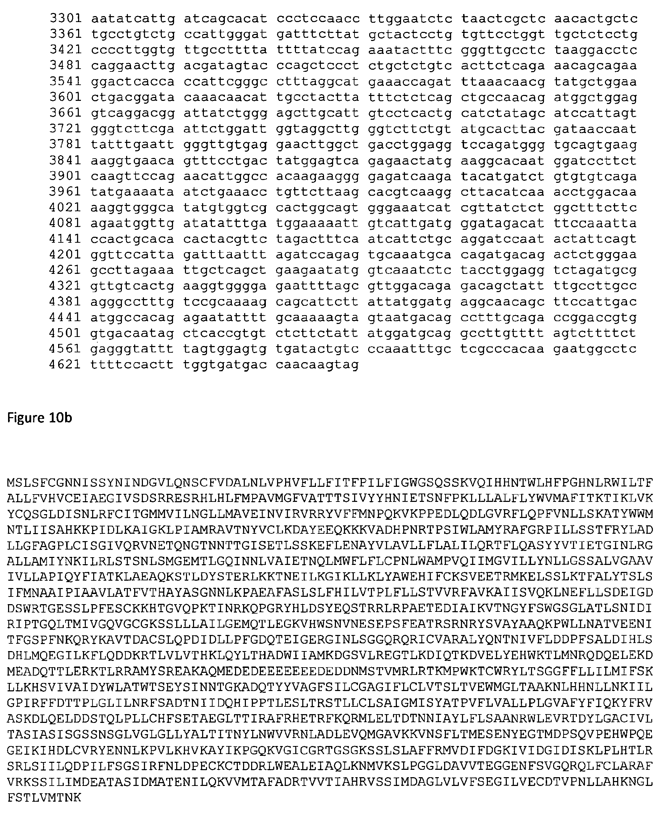

The invention relates to agents useful in the treatment of myocardial ischaemia in a male subject that would benefit from enhanced expression or function of a sulfonylurea receptor polypeptide encoded by a nucleic acid molecule selected from the group consisting of:
i) a nucleic acid molecule comprising a nucleic acid sequence as represented in Figure 10a;
ii) a nucleic acid molecule that hybridizes to the nucleic acid sequence in (i) above under stringent hybridization conditions and which encodes a polypeptide with sulfonylurea receptor activity;
wherein said agent is:
an expression vector comprising said nucleic acid molecule encoding a
sulfonylurea receptor polypeptide; wherein said nucleic acid molecule is
operably linked to at least one promoter sequence, wherein the promoter is a hypoxia inducible promoter.

There is also provided a method of diagnosis.

Muscle tissue can be of several different types and can be either skeletal, smooth or cardiac. Skeletal muscle is a voluntary muscle and its primary function is to provide means by which limbs move via tendons. Cardiac muscle is an involuntary muscle and forms the heart which functions as a pump. Smooth muscle is another example of an involuntary muscle and is typically found within the walls of organs such as the intestinal tract, bladder, and blood vessels.

The heart is an organ that functions to circulate oxgenated blood to the major organs of the body and deoxygenated blood carrying carbon dioxide to the lungs. In addition to the supply of oxygentated blood to the major organs the heart is supplied with oxygen via the coronary arteries. The heart comprises cardiac muscle which forms the walls of the heart and is an involuntary striated muscle which is adapted to be resistant to fatigue. Cardiac muscle has large numbers of mitochondria which function, amongst other things, to transduce energy enabling continuous aerobic respiration. Failure to supply sufficient oxygen to essential organs results in a condition called ischemia which can be irreversible. Myocardial ischemia is a condition that can be asymptomatic until such time as the supply of oxygen to cardiac muscle becomes restricted to the extent that the muscle fails and a heart attack occurs. Symptomatic myocardial ischemia presents as chest pain and is referred to as angina pectoris or simply "angina" which is diagnostic of a subject susceptible to heart attack.

A large number of diseases, referred to as "cardiovascular disease" can result in myocardial ischemia. For example, atherosclerosis is an inflammatory disease that results in a thickening of the walls of the arteries, especially the coronary arteries, due to plaque deposition. The plaques comprise low density lipoproteins [e.g. cholesterol and triglycerides] and macrophages and result in a narrowing ["stenosis"] of the arterial artery thereby restricting blood flow. If untreated the plaques may rupture thereby compounding the lack of blood supply to the organ. The detection of atherosclerosis is not straightforward and only angiopathy can detect significant arterial narrowing. It is not possible to detect the underlying disease and therefore it is only possible to detect atherosclerosis when the disease is quite advanced. There are some biomarkers that purport to pre-diagnose the disease [e.g. high levels of fibrinogen, elevated levels of homocysteine and/or B-type natruitetic peptide] but their clinical value is arguable. Other correlative risk factors include having diabetes, high serum levels of low-density lipoprotein, low levels of high-density lipoprotein, smoking, high blood pressure, obesity, lack of excerise and leading a sedentary life style. These are all risk factors associated with atherosclerosis some of which can be modified by altering behaviour. Further risk factors that cannot be controlled include a genetic predisposition to the disease, gender and age. Other cardiovascular diseases that can result in myocardial ischemia include hypotension [low blood pressure], hypertension [high blood pressure], thromboembolism, tumour occulsion of arteries, sickle cell anaemia, arrhythmia, cardiomyopathy, stroke, myocarditis, endocarditis, diastolic dysfunction or congestive heart disease.

Controlling diseases or conditions that result in muscle damage are known in the art. For example treatment of cardiovascular disease can either be via altering behavioural patterns or by therapeutic intervention. Altered behaviour includes stopping smoking, controlling weight and diet, and exercising. Therapeutic intervention includes both surgical and drug administration. Percutaneous transluminal coronary angioplasty is a procedure wherein a catheter which includes a balloon is inserted through the thigh which when inflated compresses the plaque to open the blocked artery. A coronary artery bypass graft is the removal of a healthy artery from, for example the leg and surigcal replacement of the diseased coronary artery. Other surgical interventions include atherectomy which effectively attempts to remove the plaques from the diseased artery. Drugs currently used to reduce the symptoms of myocardial ischemia include the use of nitrates, beta blockers and calcium channel blockers to relieve pain; the use of aspirin to prevent clot formation and the use of statins to control serum cholesterol.

An alternative approach is via gene therapy.

Gene therapy involves the transfer, and optionally the stable insertion, of new genetic information into cells. The main issues with respect to gene therapy relate to the efficient targeting of nucleic acid to cells and the establishment of high level gene expression in selected tissues. A range of viral based vectors have been used to successfully transfect mammalian cells. These include adenovirus, retrovirus, adenovirus-associated virus, papovaviruses and vaccinia virus. However, a major problem appears to be non-selective cytotoxicity, particularly in the liver, and pre-existing immune responses against the virus.

Gene therapy can involve several different approaches in the treatment of disease. The first is described as gene supplementation therapy; that is to successfully supplement defective genes that cause disease with wild type copies delivered to target cells, for example as in the case of cystic fibrosis. The second is "cell factory" gene therapy, which uses DNA or RNA as a therapeutic for expression in cells to treat deficiencies such as diabetes. In all cases successful delivery of the gene therapy vector (which can be viral or synthetic) would be improved if the vector had selectivity for binding and entry into target cells.

Gene therapy in the treatment of cardiovascular disease is known in the art. For example, WO2008/054713 describes a gene therapy for the treatment of cardiovascular disease by expression of a nucleic acid encoding a polypeptide that has Ca²⁺ sensing activity [protein S100A1] which is abnormal in heart failure and cardiovascular disease. WO2008/013692 also discloses gene therapy in the treatment of cardiovascular disease by direct epicardial infusion of expression vectors comprising nucleic acids encoding a sarcoplasmiclendoplasmic reticulum ATPase to regulate Ca²⁺ homeostasis in the heart. A further example is described in WO2005/047473 which discloses the expression of both cycloxygenase and prostacyclin synthase in the control of primary and secondary hypertension or vascular disease. The use of gene therapy in the protection of subjects susceptible to myocardial infarction is described in US2002/0061299 through the expression of antioxidant enzymes. Gene therapy is also an approach to control congestive heart failure. For example, WO01/48164 describes the insertion of transgenes into the myocardium to increase beta-andrenegic responsiveness. Gene therapy in the treatment of skeletal and smooth muscle conditions is also known in the art. For example, US2008/0069803 describes adeno-associated virons and their use in delivering therapeutic proteins to muscle tissue by intramuscular injection. US2006/0148740 discloses gene therapy vectors useful in the treatment of the muscle wasting disease Duchenne Muscular Dystrophy. The treatment of smooth muscle conditions such as erectile dysfunction and bladder conditions by gene therapy is described in US2007/0086982. It is apparent that means exist for the delivery and specific expression of a therapeutic protein to cardiac, skeletal and smooth muscle. Scientific article - Overexpression of SUR2A generates a cardiac phenotype resistant to inchemia authored by Du Qingyou et al. (Faseb Journal, vol.20, no.8, June 2006, pages 1131 to 1141, XP002556068) discloses SUR2A under the control of a cytomegalovirus promoter and its use in regulating cardiac resistance to ischaemia.

The prior art also includes J. Physiol 564.2, 2005, 619-630. This discloses:a) Male and female rats were exercised. Perfused rat hearts were then subjected to ischaemia and reperfusion. Short term exercise was found to be infarct sparing in both sexes; acquisition of protection against infarction was demonstrated as more rapid in males than females (page 625, penultimate paragraph). b) Infarct sparing effects were found to correlate with increased SUR2A protein expression (page 628, table 3); infarct sparing effects and corrrelation with increased SUR2A expression was more rapid in males (after 1 day of exercise as compared to only being seen after 5 days with females).

This disclosure relates to the manipulation of expression of the sulfonylurea receptor 2A (SUR2A) which belongs to the group of atypical ABC proteins known to regulate the activity of ATP-sensitive K⁺ channels in the heart. We describe agents that lead to overexpression of SUR2A and therapeutic agents for use in the treatment of myocardial ischaemia in a male subject. We also describe a method to diagnose a male subject suffering from or having a predisposition to myocardial ischemia.

According to an aspect of the invention there is provided a therapeutic agent for use in the treatment of myocardial ischaemia in a male subject that would benefit from enhanced expression or function of a sulfonylurea receptor polypeptide encoded by a nucleic acid molecule selected from the group consisting of:
i) a nucleic acid molecule comprising a nucleic acid sequence as represented in Figure 10a;
ii) a nucleic acid molecule that hybridizes to the nucleic acid sequence in (i) above under stringent hybridization conditions and which encodes a polypeptide with sulfonylurea receptor activity;
wherein said agent is:
an expression vector comprising said nucleic acid molecule encoding a sulfonylurea receptor polypeptide; wherein said nucleic acid molecule is operably linked to at least one promoter sequence, wherein the promoter is a hypoxia inducible promoter.

Hybridization of a nucleic acid molecule occurs when two complementary nucleic acid molecules undergo an amount of hydrogen bonding to each other. The stringency of hybridization can vary according to the environmental conditions surrounding the nucleic acids, the nature of the hybridization method, and the composition and length of the nucleic acid molecules used. Calculations regarding hybridization conditions required for attaining particular degrees of stringency are discussed in Sambrook et al., Molecular Cloning: A Laboratory Manual (Cold Spring Harbor Laboratory Press, Cold Spring Harbor, NY, 2001); and Tijssen, Laboratory Techniques in Biochemistry and Molecular Biology-Hybridization with Nucleic Acid Probes Part I, Chapter 2 (Elsevier, New York, 1993). The Tₘ is the temperature at which 50% of a given strand of a nucleic acid molecule is hybridized to its complementary strand. The following is an exemplary set of hybridization conditions and is not limiting:

| Very High Stringency (allows sequences that share at least 90% identity to hybridize) | |
|---|---|
| Hybridization: | 5x SSC at 65°C for 16 hours |
| Wash twice: | 2x SSC at room temperature (RT) for 15 minutes each |
| Wash twice: | 0.5x SSC at 65°C for 20 minutes each |

| High Stringency (allows sequences that share at least 80% identity to hybridize) | |
|---|---|
| Hybridization: | 5x-6x SSC at 65°C-70°C for 16-20 hours |
| Wash twice: | 2x SSC at RT for 5-20 minutes each |
| Wash twice: | 1x SSC at 55°C-70°C for 30 minutes each |

| Low Stringency (allows sequences that share at least 50% identity to hybridize) | |
|---|---|
| Hybridization: | 6x SSC at RT to 55°C for 16-20 hours |
| Wash at least twice: | 2x-3x SSC at RT to 55°C for 20-30 minutes each. |

In the disclosure made said sulfonylurea receptor polypeptide comprises or consists of an amino acid sequence as represented in Figure 10b, or a variant amino acid sequence wherein said variant sequence varies from the amino acid sequence in Figure 10b by the addition deletion or substitution of at least one amino acid residue wherein said variant has sulfonylurea receptor activity.

A variant polypeptide may differ in amino acid sequence by one or more substitutions, additions, deletions, truncations that may be present in any combination. Among preferred variants are those that vary from a reference polypeptide by conservative amino acid substitutions. Such substitutions are those that substitute a given amino acid by another amino acid of like characteristics. The following non-limiting list of amino acids are considered conservative replacements (similar): a) alanine, serine, and threonine; b) glutamic acid and aspartic acid; c) asparagine and glutamine d) arginine and lysine; e) isoleucine, leucine, methionine and valine and f) phenylalanine, tyrosine and tryptophan. Most highly preferred are variants that retain or enhance the same biological function and activity as the reference polypeptide from which it varies. Variant polypeptides include natural polymorphic variants that exist.

In addition, the disclosure features polypeptide sequences having at least 60-75% identity with the polypeptide sequences as herein disclosed, or fragments and functionally equivalent polypeptides thereof. In one embodiment, the polypeptides have at least 85% identity, more preferably at least 90% identity, even more preferably at least 95% identity, still more preferably at least 97% identity, and most preferably at least 99% identity with the amino acid sequences illustrated herein.

Said agent is an expression vector comprising the nucleic acid molecule of figure 10a encoding a sulfonylurea receptor polypeptide; wherein said nucleic acid molecule is operably linked to at least one promoter sequence, wherein the promoter is a hypoxia inducible promoter.

"Promoter" is an art recognised term and, for the sake of clarity, includes the following features which are provided by example only. Enhancer elements are *cis* acting nucleic acid sequences often found 5' to the transcription initiation site of a gene (enhancers can also be found 3' to a gene sequence or even located in intronic sequences). Enhancers function to increase the rate of transcription of the gene to which the enhancer is linked. Enhancer activity is responsive to *trans* acting transcription factors which have been shown to bind specifically to enhancer elements. The binding/activity of transcription factors (please see Eukaryotic Transcription Factors, by David S Latchman, Academic Press Ltd, San Diego) is responsive to a number of physiological/environmental cues.

Promoter elements also include so called TATA box and RNA polymerase initiation selection sequences which function to select a site of transcription initiation. These sequences also bind polypeptides which function, *inter alia,* to facilitate transcription initiation selection by RNA polymerase.

Adaptations also include the provision of selectable markers and autonomous replication sequences which facilitate the maintenance of said vector in either the eukaryotic cell or prokaryotic host. Vectors which are maintained autonomously are referred to as episomal vectors. Episomal vectors are desirable since these molecules can incorporate large DNA fragments (30-50kb DNA). Episomal vectors of this type are described in WO98/07876.

Adaptations which facilitate the expression of vector encoded genes include the provision of transcription termination/polyadenylation sequences. This also includes the provision of internal ribosome entry sites (IRES) which function to maximise expression of vector encoded genes arranged in bi-cistronic or multi-cistronic expression cassettes. Expression control sequences also include so-called Locus Control Regions (LCRs).. These are regulatory elements which confer position-independent, copy number-dependent expression to linked genes when assayed as transgenic constructs. LCRs include regulatory elements that insulate transgenes from the silencing effects of adjacent heterochromatin, Grosveld et al., Cell (1987), 51: 975-985.

There is a significant amount of published literature with respect to expression vector construction and recombinant DNA techniques in general. Please see, Sambrook et al (1989) Molecular Cloning: A Laboratory Manual, Cold Spring Harbour Laboratory, Cold Spring Harbour, NY and references therein; Marston, F (1987) DNA Cloning Techniques: A Practical Approach Vol III IRL Press, Oxford UK; DNA Cloning: F M Ausubel et al, Current Protocols in Molecular Biology, John Wiley & Sons, Inc. (1994).

The use of viruses or "viral vectors" as therapeutic agents is well known in the art. Additionally, a number of viruses are commonly used as vectors for the delivery of exogenous genes. Commonly employed vectors include recombinantly modified enveloped or non-enveloped DNA and RNA viruses, preferably selected from *retroviridae baculoviridiae, parvoviridiae, picornoviridiae, herpesveridiae, poxviridae, adenaviridiae, or picornnaviridiae.* Chimeric vectors may also be employed which exploit advantageous elements of each of the parent vector properties (See e.g., Feng, et al. (1997) Nature Biotechnology 15:866-870). Such viral vectors may be wild-type or may be modified by recombinant DNA techniques to be replication deficient, conditionally replicating or replication competent.

Preferred vectors are derived from retroviral genomes [e.g. lentivirus].

Viral vectors may be conditionally replicating or replication competent. Conditionally replicating viral vectors are used to achieve selective expression in particular cell types while avoiding untoward broad spectrum infection. Examples of conditionally replicating vectors are described in Pennisi, E. (1996) Science 274:342-343; Russell, and S.J. (1994) Eur. J. of Cancer 30A(8):1165-1171. Additional examples of selectively replicating vectors include those vectors wherein a gene essential for replication of the virus is under control of a promoter which is active only in a particular cell type or cell state such that in the absence of expression of such gene, the virus will not replicate. Examples of such vectors are described in Henderson, et al., United States Patent No. 5,698,443 issued December 16, 1997 and Henderson, et al.; United States Patent No. 5,871,726 issued February 16, 1999.

Additionally, the viral genome is modified to include a hypoxia inducible promoters which achieves replication or expression only under certain conditions Examples of inducible promoters are known in the scientific literature (See, e.g. Yoshida and Hamada (1997) Biochem. Biophys. Res. Comm. 230:426-430; lida, et al. (1996) J. Virol. 70(9):6054-6059; Hwang, et al. (1997) J. Virol 71(9):7128-7131; Lee, et al. (1997) Mol. Cell. Biol. 17(9):5097-5105; and Dreher, et al. (1997) J. Biol. Chem 272(46); 29364-29371.

Viral vectors are delivered intravenously, intrarterial, intrapericardial or intramuscularly as well as direct injection into heart or skeletal muscle.

Preferably said hypoxia inducible promoter is selected from the group consisting of: Adenosine A2B receptor (A2BR) promoter, Plasminogen activator receptor (uPAR) promoter, VEGF receptors (VEGFR1 and VEGFR2) promoter, Platelet-derived endothelial cell growth factor/thymidine phosphorylase (PDECGF/TP) promoter, nitric oxide synthase (NOS) promoter, Phosphoglycerate kinase-1 (PGK-1) promoter, Pyruvate kinase M (PK-M) promoter, Glucose transporter 1 (GLUT1) promoter, Hypoxia-inducible factor (HIF-1) promoter, Early growth response 1 (Egr-1) promoter, Nuclear factor kB (NFkB) promoter, Hepatocyte growth factor activator (HGFA) promoter, Vascular endothelial growth factor (VEGF) promoter, CXCL8 promoter, CCL11 promoter, Transforming growth factor-beta (TGF-β) promoter, procollagen promoter, Integrin-linked kinase (ILK) promoter, KIPDC1 promoter, Erythropoietin (EPO) promoter, Serine/Threonine Kinase-15 (STK15) promoter, the histone demethylase Jumonji domain containing 1A (JMJD1A) promoter, Endothelin-2 (EDN2) promoter, choline kinase (Chk) promoter, Sphingosine kinase 1 promoter, Carcinoembryonic antigen (CEA, ceacam5) promoter, Monocyte chemoattractant protein-1 (MCP-1/CCL2) promoter, MCP-5 (Ccl12) promoter, prostate-specific antigen (PSA) promoter, c-Met promoter, Matrix metalloproteinases Class III beta-tubulin (TUBB3) promoter, Glutamine: fructose-6-phosphate amidotransferase (GFAT) promoter, Protein phosphatase 1 nuclear targeting subunit beta-secretase (BACE1) promoter or Plasminogen activator inhibitor-1 (PAI-1) promoter.

In an alternative preferred embodiment of the invention said hypoxia inducible promoter is a promoter that controls the expression of a mitochondrial protein.

In a preferred embodiment of the invention SUR2A is provided with a mitochondrial targeting sequence that targets SUR2A to mitochondria.

In a preferred embodiment of the invention said mitochondrial targeting sequence is derived from a mitochondrial protein selected from the group consisting of: anine/glyoxylate aminotransferase 1 (AGT), pyruvate dehydrogenase E1 alpha gene (PDH E1), manganese superoxide dismutase (MnSOD), Bax, mitochondrial protein kinase Cepsilon (PK Cepsilon), Bfl1, HCCS-1, translocase of outer membrane 22 (tom22), restorer gene (Rf-1A), trypanosoma cruzi (TcBPP1), a novel splice variant of the neuronal Bbeta regulatory subunit (Bbeta2), cAMP-dependent protein kinase anchoring protein (d-AKAP1), beta-naphthoflavone-inducible cytochrome (P4501A1) p13Il, a new human half-molecule ABC protein, designated M-ABC1, vesicle-associated membrane protein-1 (VAMP-1), adenine nucleotide translocator (ANT), methionine sulfoxide reductase (MSRA), Human Factor B, Oxygen-regulated protein 150 (ORP150) Peroxiredoxin V (PRDX5), Protein tyrosine phosphatase interacting protein 51 (PTPIP51), cytochrome P4502B1, cytochrome P4501A1, The DNA helicase, Hmi1p A-kinase-anchoring protein 1 (D-AKAP1), Tim23p attached to a constitutive promoter.

In preferred embodiment of the invention and the mitochondrial targeting sequence is (MSVLTPLLLRGLTGSARRLPVPRAKIHSL).

In a preferred embodiment of the invention said mitochondrial promoter is the light or heavy strand mitochondrial DNA promoter.

In a further preferred embodiment of the invention said agent is combined with a second different therapeutic agent.

In a preferred embodiment of the invention said agent is an anti-inflammatory agent effective in the treatment of ischemia.

Preferably said anti-inflammatory agent is selected from the group consisting of: aspirin, salsalate, diflunisal, ibuprofen, ketoprofen, nabumetone, piroxicam, naproxen, dicolfenac, indomethacin, sulindac, tolmetin, etodolac, ketoralac, oxaprozin or celecoxib.

In an alternative preferred embodiment of the invention said second agent is a statin.

Preferably said statin is selected from the group consisting of: atorvastatin, cerivastatin, fluvastatin, lovastatin, mevastatin, pitavastatin, rousuvastatin or simvastatin.

In an alternative preferred embodiment of the invention said second agent is a antaplatelet agent effective in the treatment of ischaemia.

Preferably said antiplatelet agent is selected from the group consisting of: aspirin, ticlopidine, clopidogrel, cilostazol, dypiradomole.

In a preferred embodiment of the invention said agent is a β andrenergic blocker; preferably said β andrenergic blocker is selected from the group consisting of: propranolol, acebutalol, atenolol, bisoprolol, metoprolol, nadolol, timolol, nebivolol.

In an alternative preferred embodiment of the invention said agent is a calcium channel antagonist; preferably said antagonist is selected from the group consisting of: nisoldipine, nifedipine,nicardipine, bepridil, isradipine, nimodipine, felodipine, nimodipine, amlodipine, diltiazem, verapamil.

The compositions of the invention are administered in effective amounts. An "effective amount" is that amount of a composition that alone, or together with further doses, produces the desired response. Such amounts will depend, of course, on the particular condition being treated, the severity of the condition, the individual patient parameters including age, physical condition, size and weight, the duration of the treatment, the nature of concurrent therapy (if any), the specific route of administration and like factors within the knowledge and expertise of the health practitioner. These factors are well known to those of ordinary skill in the art and can be addressed with no more than routine experimentation. It is generally preferred that a maximum dose of the individual components or combinations thereof be used, that is, the highest safe dose according to sound medical judgment. It will be understood by those of ordinary skill in the art, however, that a patient may insist upon a lower dose or tolerable dose for medical reasons, psychological reasons or for virtually any other reasons.

The desired response in treating myocardial ischemia is the symptomatic treatment of the consequences of the disease. This may involve only the partial improvement of the symptomatic consequences of the disease, although more preferably, it involves complete improvement of the symptomatic consequences of the disease. This can be monitored by routine methods.

More particularly improvements in myocardial ischemia can be monitored by any one of the following indicia:
1 Tolerance to exercise and physical stress
2 Exercise stress ECG testing
3 Heart function on echocardiography
4 ECG signs of ischaemia

The pharmaceutical compositions used in the foregoing methods preferably are suitable for oral administration and contain an effective amount of an agent according to the invention for producing the desired response in a unit of weight or volume suitable for administration to a patient. The response can, for example, be measured by determining decrease of disease symptoms.

The doses of the agent according to the invention administered to a subject can be chosen in accordance with different parameters, in particular in accordance with the mode of administration used and the state of the subject. Other factors include the desired period of treatment or a subject's body mass index. In the event that a response in a subject is insufficient at the initial doses applied, higher doses (or effectively higher doses by a different, more localized delivery route) may be employed to the extent that patient tolerance permits.

Other protocols for the administration of agents will be known to one of ordinary skill in the art, in which the dose amount, schedule and mode of administration and the like vary from the foregoing. The administration of compositions to mammals other than humans, (e.g. for testing purposes or veterinary therapeutic purposes), is carried out under substantially the same conditions as described above. A subject, as used herein, is a mammal, preferably a human, and including a non-human primate, cow, horse, pig, sheep, goat, dog, cat or rodent.

When administered, the agents of the invention are applied in pharmaceutically-acceptable amounts and in pharmaceutically-acceptable compositions. The term "pharmaceutically acceptable" means a non-toxic material that does not interfere with the effectiveness of the biological activity of the active ingredients. Such preparations may routinely contain salts, buffering agents, preservatives, compatible carriers, and optionally other therapeutic agents. When used in medicine, the salts should be pharmaceutically acceptable, but non-pharmaceutically acceptable salts may conveniently be used to prepare pharmaceutically-acceptable salts thereof and are not excluded from the scope of the invention. Such pharmacologically and pharmaceutically-acceptable salts include, but are not limited to, those prepared from the following acids: hydrochloric, hydrobromic, sulphuric, nitric, phosphoric, maleic, acetic, salicylic, citric, formic, malonic, succinic, and the like. Also, pharmaceutically-acceptable salts can be prepared as alkaline metal or alkaline earth salts, such as sodium, potassium or calcium salts. The compositions also may contain, optionally, suitable preservatives, such as: benzalkonium chloride; chlorobutanol; parabens and thimerosal. The agents of the invention can exist in different forms, such as acids, esters, salts and tautomers, for example, and the invention includes all variant forms of the agents.

Compositions may be combined, if desired, with a pharmaceutically-acceptable carrier. The term "pharmaceutically-acceptable carrier" as used herein means one or more compatible solid or liquid fillers, diluents or encapsulating substances which are suitable for administration into a human. The term "carrier" in this context denotes an organic or inorganic ingredient, natural or synthetic, with which the active ingredient is combined to facilitate the application, [e.g. liposome based]. The components of the pharmaceutical compositions also are capable of being co-mingled with the molecules of the present invention, and with each other, in a manner such that there is no interaction which would substantially impair the desired pharmaceutical efficacy.

The compositions may conveniently be presented in unit dosage form and may be prepared by any of the methods well-known in the art of pharmacy. All methods include the step of bringing the active agent into association with a carrier which constitutes one or more accessory ingredients. In general, the compositions are prepared by uniformly and intimately bringing the active compound into association with a liquid carrier, a finely divided solid carrier, or both, and then, if necessary, shaping the product.

Compositions suitable for oral administration may be presented as discrete units, such as capsules, tablets, lozenges, each containing a predetermined amount of the active compound. Other compositions include suspensions in aqueous liquids or non-aqueous liquids such as syrup, elixir or an emulsion or as a gel. Compositions may be administered as aerosols and inhaled.

Compositions suitable for parenteral administration conveniently comprise a sterile aqueous or non-aqueous preparation of agent which is preferably isotonic with the blood of the recipient. This preparation may be formulated according to known methods using suitable dispersing or wetting agents and suspending agents. The sterile injectable preparation also may be a sterile injectable solution or suspension in a non-toxic parenterally-acceptable diluent or solvent, for example, as a solution in 1, 3-butane diol. Among the acceptable solvents that may be employed are water, Ringer's solution, and isotonic sodium chloride solution. In addition, sterile, fixed oils are conventionally employed as a solvent or suspending medium. For this purpose any bland fixed oil may be employed including synthetic mono-or di-glycerides. In addition, fatty acids such as oleic acid may be used in the preparation of injectables. Carrier formulation suitable for oral, subcutaneous, intravenous, intramuscular, etc. administrations can be found in Remington's Pharmaceutical Sciences, Mack Publishing Co., Easton, PA.

In a preferred emodiment of the invention said subject is healthy.

In a preferred embodiment of the invention said subject has a predisposition to or is suffering from myocardial ischemia.

Said subject is male.

In a preferred embodiment of the invention said subject is elderly.

In a preferred embodiment of the invention said subject is at least 60 years old. More preferably said subject is 60-80 years old; preferably said subject is greater than 80 years old.

In a preferred embodiment of the invention said subject suffers from a disease or condition that causes fatigue.

In a preferred embodiment of the invention said subject suffers from a disease or condition selected from the group consisting of: chronic obstructive pulmonary disease, heart failure, coronary artery spasm, dissecting aneurysms, infectious vasculitis, congenital defects in the coronary vascular system and pulmonary hypertension wherein the disease indirectly results in myocardial ischemia.

According to a further aspect of the invention there is provided a method to diagnose a male subject suffering from or having a predisposition to myocardial ischemia comprising:
(i) determining the expression of SUR2A mRNA or protein in a blood or serum sample obtained from a male patient;
(ii) comparing the expression of SUR2A in said sample to a control sample wherein if the expression of SUR2A in said sample is lower than the expression of SUR2A in the control, the subject may benefit from administration of an agent according to anv one of claims 1 to 5: and
(iii) determining whether said subject would benefit from administration of an agent according to any one of claims 1 to 5.

In a further preferred method of the disclosure said subject is administered an agent according to the invention.

Throughout the description and claims of this specification, the words "comprise" and "contain" and variations of the words, for example "comprising" and "comprises", means "including but not limited to", and is not intended to (and does not) exclude other moieties, additives, components, integers or steps.

Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics, compounds, chemical moieties or groups described in conjunction with a particular aspect, embodiment or example of the invention are to be understood to be applicable to any other aspect, embodiment or example described herein unless incompatible therewith.

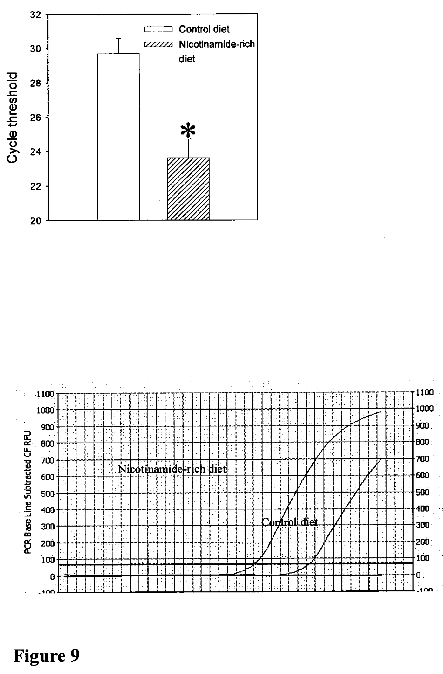

An embodiment of the invention will now be described by example only and with reference to the following figures:

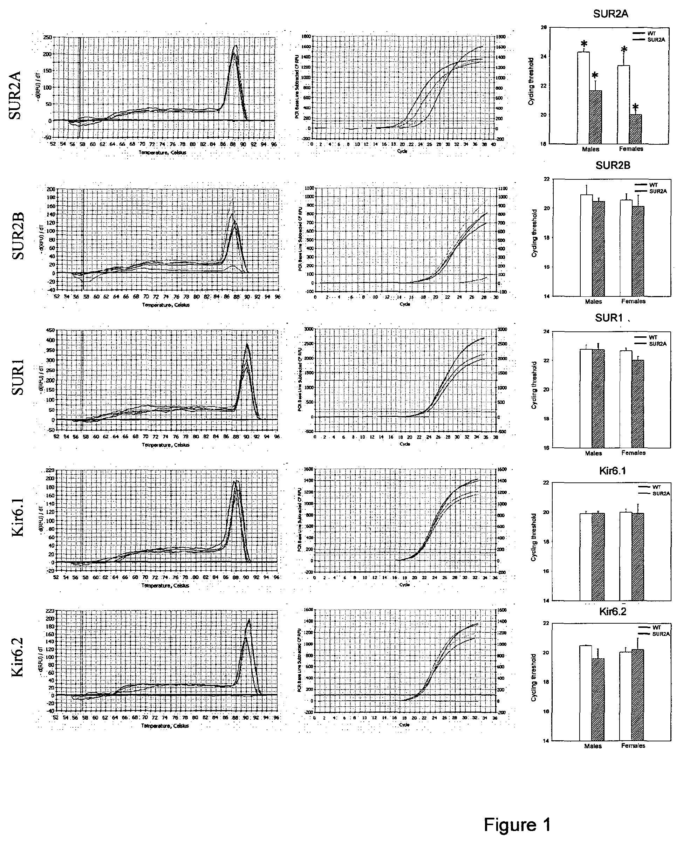

Figure 1 illustrates levels of mRNA of K_{ATP} channel subunits in the wild type/transgene, male/female mouse hearts. Melting curve analysis of SUR2A, SUR2B, SUR1, Kir6.1 and Kir6.2 amplicons (graphs on the left). Shown are plots of fluorescence (-dF1/dT) versus temperature with different amounts of cDNA template (40 pg, 200 pg, 1 ng. 5 ng, 25 ng) done in duplicates (graphs on the left). The presence of a single peak is consistent with the formation of a single amplicon and indicates the lack of primer-dimer formation. Representative progress curves for the real-time PCR amplification of SUR2A, SUR2B, SUR1, Kir6.1 and Kir6.2 SUR2A and Kir6.2 cDNA in male and female wild type and transgenic mice (middle graphs). Bar graphs corresponding to the progress curves. Each bar represents mean±SEM (n=6 for each). *P<0.05 when compared to each other.
Figure 2 illustrates resistance of male and female wild type and transgenic hearts to ischaemia-reperfusion. Typical photographs of myocardial slices from male and female wild type and transgenic mice exposed to ishaemia-reperfusion. Infarcted areas are pale/grey while viable myocardium is dark/red. Bar graphs depict myocardial infarct size expressed as a percentage of area at risk zone (n=5-9). *P<0.05.
Figure 3 illustrates resistance of cardiomyocytes from male and female wild type and transgenic mice to hypoxia. Laser confocal images of exposed to 30 min-long hypoxia (magnification was x40). Bar graph depicts percentage of cells that died/survived, n=14-28. *P<0.01.
Figure 4 illustrates mitochondrial membrane potential in cardiomyocytes from male and female wild type and transgenic mice. Original images of cardiomyocytes loaded with JC-1 and corresponding graph under depicted conditions. Each bar represent mean±S.E.M (n=4-7). *P<0.05 when compared to all other groups.
Figure 5 illusrates mitochondrial membrane potential in cardiomyocytes during diastole and systole. Original images of cardiomyocytes loaded with JC-1 and corresponding graph under depicted conditions. Each bar represent mean±S.E.M (n=12).
Figure 6 illustrates western blot with anti-SUR2 antibody of immunoprecipitate from cardiac mitochondrial fractions obtained from male and female wild type (WT) and transgenic (SUR2A) mice.
Figure 7 illustrates hypoxia and mitochondrial membrane potential in cardiomyocytes from male and female wild type and transgenic mice. Original images of cardiomyocytes loaded with JC-1 under hypoxia and corresponding bar and line-scatter graphs.
Figure 8 illustrates mitochondrial membrane potential in cardiomyocytes from male and female wild type and transgenic mice at the end of 30 min-long hypoxia. Original images of cardiomyocytes loaded with JC-1 and corresponding graph under depicted conditions. Each bar represent mean±S.E.M (n=4-7). *P<0.05 when compared to all other groups.
Comparative Figure 9 illustrates that a nicotinamide-rich diet stimulates expression of SUR2A. Representative progress curves for the real-time PCR amplification of SUR2A cDNA. Bar graphs showing cycle threshold for the real-time PCR amplification of SUR2A. Each bar represents mean±SEM (n=6 for each). Methods of real time RT-PCR (primers etc.) is described in details in our paper Du et al., 2006, FASEB J 20: 1131-1141. *P=0.005.
Figure 10a is the nucleic acid sequence of SUR2A; Figure 10b is the amino acid sequence of human SUR2A;

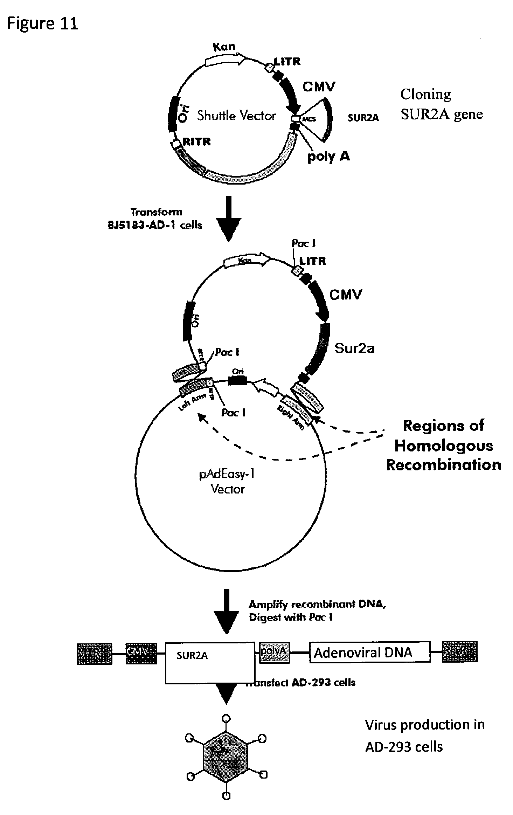

Figure 11 illustrates the production of recombinant SUR2A adenovirus using the Adeasy XL adenoviral vector system;

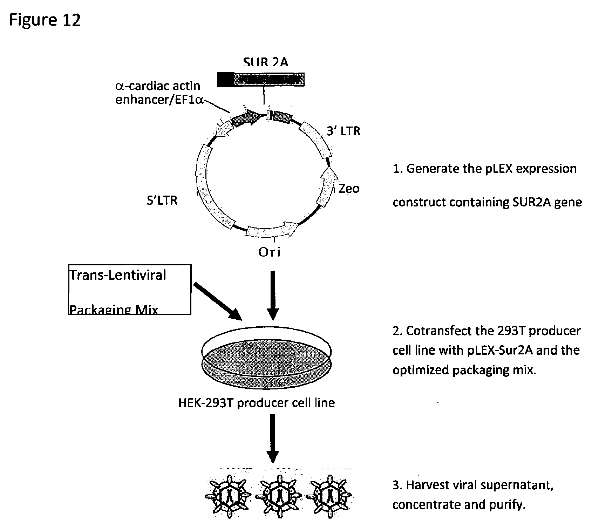

Figure 12 illustrates the production of SUR2A lentovirus using Trans-Lentiviral™ ORF Packaging System;

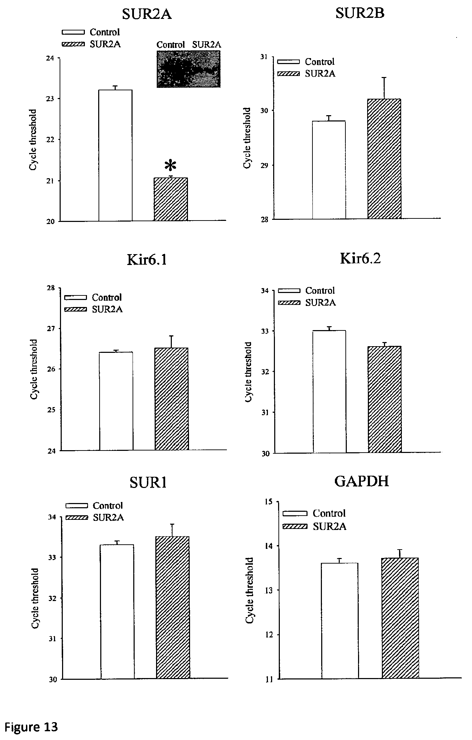

Figure 13. Infection with AV-SUR2A increases expression of SUR2A without affecting the expression of other K_{ATP} channel subunits. Bar graphs represent cycling thresholds of the real time RT-PCR progress curves of K_{ATP} channel-forming subunits. Inset in SUR2A graph is a Western blot of anti-Kir6.2 immunoprecipitate from H9C2 cells with anti-SUR2 antibody under depicted conditions. Each bar represents mean±SEM (n=4 for each). *P<0.05.

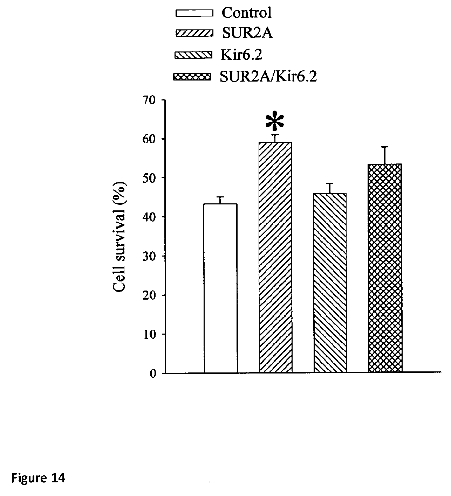

Figure 14. Infection with AV-SUR2A increases survival of cells exposed to DNP. A bar graph showing a percentage of survival in control cells and cells infected with SUR2A alone, Kir6.2 alone or SUR2A/Kir6.2 exposed to DNP (10 mM). Each bar represent mean±SEM (n=3-14). *P<0.05 when compared to Kir6.2AFA and ⁺P<0.05 when compared to SUR2A/Kir6.2AFA;

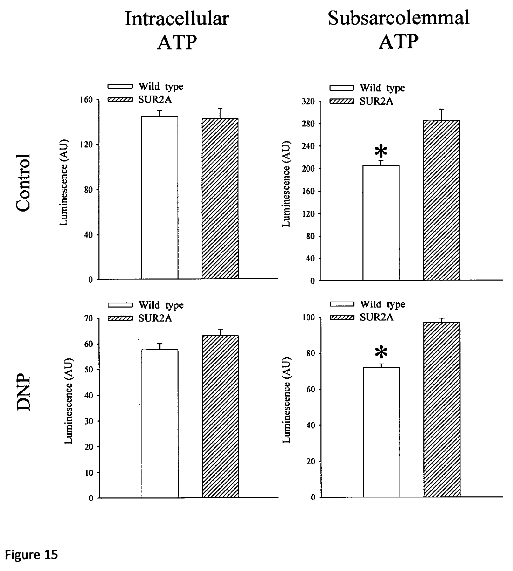

Figure 15. infection with AV-SUR2A increases the levels of subsarcolemmal ATP. Bar graphs showing luciferase (intracellular ATP) and annexin-luciferase (subsarcolemmal ATP) luminescence in control cells and cells infected with AV-SUR2A under control conditions (control) and after treatment with 10 mM DNP (DNP). Each bar represent mean±SEM (n=5). *P<0.05; and

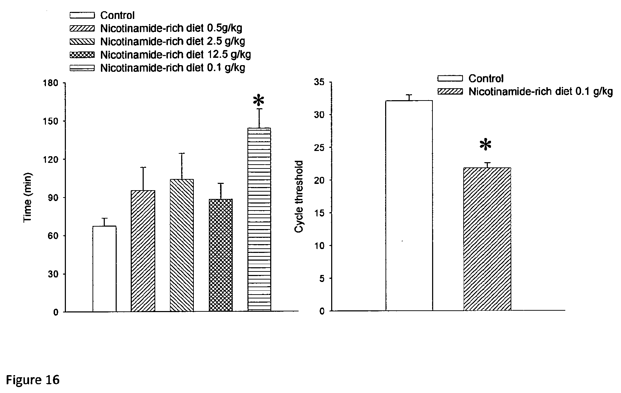

Comparative Figure 16. Nicotinamide increases physical endurance and expression of SUR2A. Duration of stay on treadmill (graph on the lef) and expression of SUR2A (graph on the right) in hearts of mice on control and nicotinamide-rich diet. Each bar represents mean±SEM (n=6). *P<0.01.

### MATERIALS AND METHODS

### SUR2A mice

All experiments have been done on male and female mice overexpressing SUR2A (SUR2A mice) and their littermate controls. Generation, breeding and genotyping of these mice have previously been described in detail [14].

### Real time RT-PCR

Total RNA was extracted from cardiac ventricular tissue of transgenic and wild type mice using TRIZOL reagent (Invitrogen, Paisley, UK) according to the manufacturer's recommendations. Extracted RNA was further purified with RNeasy Mini Kit (Qiagen, Crawley, UK) according to the manufacturer's instruction. The specific primers for mouse SUR2A, Kir6.2, Kir6.1, SUR1 and SUR2B (designed to amplify SUR2B and natively present SUR2A) were designed as follows: For SUR2A: sense, ACTATGGAGTCCGAGAACTA, antisense, AGGTTTGGACCAGTATCACA; for Kir6.2: sense; ACATGCAGGTGGTGCGCAÀG, antisense, AGGGCATCCAGCAGACTGCG; for Kir6.1: sense, 5'-GTCACACGCTGGTCATCTTCAC-3, antisense, 5-GGCATTCCTCAGTCATCATTCTCC-3'; for SUR1: sense, 5'-GGAAGGACTCACCACCATC-3', antisense, 5'-GAGACCATCAAGGCGTAGG-3'; for SUR2B: sense, 5'-GATGCCACTGTCACCGAAG-3', antisense, 5-TCATCACAATAACCAGGTCTGC-3'. The real time RT-PCR was performed as described in ref. 14.

### Heart collection and ischaemia-reperfusion injury

Mice were sacrificed by cervical dislocation (according to UK Home Office procedures), weighed immediately and the heart dissected into ice-cold Tyrode's solution (pH 7.4). The aorta was then cannulated and the heart attached to a Langendorff perfusion apparatus where it was perfused with 37°C oxygenated Tyrode's solution for a stabilisation period of 70 minutes. The heart was then subjected to 30 minutes of ischaemia by placing it into degassed Tyrode's and switching off perfusion. A second 30 minute reperfusion with oxygenated Tyrode's followed the ischaemia. After reperfusion, hearts were snap-frozen in liquid nitrogen and stored at -80 °C. The frozen heart was divided into approximately 5-6 transverse sections, which were weighed before staining for 1 hour in 10% Triphenyltetrazolium chloride (TTC) in Phosphate Buffer Saline (PBS-both Sigma-Aldrich, Dorset, UK) at 37 °C. The stain was fixed in 4% Paraformaldehyde (Sigma-Aldrich) for 30 minutes, following which, the tissue was photographed and the area of infarcted tissue measured using Image Analysis software [14].

### Immunoprecipitation and Western blotting analysis

Sheep anti-SUR2 antibody was used for immunoprecipitation and Western blotting in this study (described in detail in ref. 15). To obtain the mitochondrial cardiac fraction, mouse ventricular tissue was homogenised in buffer I (TRIS 10 mM, NaH₂PO₄ 20 mM, EDTA 1 mM, PMSF 0.1 mM, pepstatin 10 □g/ml, leupeptin 10 □g/ml, at pH=7.8) and incubated for 20 min (at 4°C). The osmolarity was restored with KCI, NaCl and sucrose and the obtained mixture was centrifuged at 500 g. The supernatant was diluted in buffer II (imidazole 30 mM, KCI 120 mM, NaCl 30 mM, NaH₂PO₄ 20 mM, sucrose 250 mM, pepstatin 10 µg/mi, leupeptin 10 □g/ml, at pH=6.8) and centrifuged at 7000 *g* for 15 min to isolate mitochondria (the obtained pellet contains the mitochondrial fraction). Protein concentration in resuspended pellet was determined using Bradford method. 40 µg of the epitope-specific SUR2 antibody was pre-bound to Protein-G Sepharose beads and used to immunoprecipitate from 50 µg of mitochondrial fraction protein extract. The pellets of this precipitation were run on SDS polyacrylamide gels for Western analysis. Western blot probing was performed using 1/1000 dilutions of anti-SUR2 antibody and detection was achieved using Protein-G HRP and ECL reagents. The band intensities were analyzed using the Quantiscan software.

### lsolafion of single cardiomyocytes

Ventricular cardiomyocytes were dissociated from the mouse using an established enzymatic procedure [14, 15].

### Experimental protocol of cellular hypoxia.

Hypoxia of isolated cardiomyocytes has been performed as described [14, 15]. Thus, cardiomyocytes were placed into Tyrode's solution (in mM: NaCl 136.5, KCI 5.4, CaCl2 1.8, MgCl₂ 0.53, glucose 5.5, HEPES-NaOH 5.5, pH 7.4), plated out on glass coverslips and paced to beat by field stimulation (parameters of the stimulation: 5-20 mV depending on cellular threshold, 5 ms, 1 Hz). Beating cardiomyocytes were perfused with Tyrode solution at a rate of 3 ml/min and, under these conditions, the partial pressure of O₂ (PO₂ in perfusate was 140 mmHg. To induce hypoxia, Tyrode solution was bubbled with 100% argon (PO₂=20 mm Hg).

### Laser confocal microscopy

Mitochondrial membrane potential was monitored in cells exposed to hypoxia as described [15, 16]. To measure the mitochondrial membrane potential cells were loaded with JC-1 according to the manufacturer's instructions (Invitrogen, Paisley, UK) and continously monitored with LSM-510. Fluorescence was imaged at 488 nm excitation wavelength and emission was captured at 530 and 590 nm for green and red channels respectively; the JC-1 ratio refers always to green/red ratio. The moment of cell death was defined as the point when the cell has become rounded (ratio of diameters <2). In addition to mitochondrial membrane potential, intracellular Ca²⁺ levels were also measured using Fluo-3, Ca²⁺-sensitive dye [described in 11-13].

### H9C2 cells and viral constructs

H9C2 cells Rat embryonic heart H9c2 cells (ECACC, Salisbury, UK) were cultured in a tissue flask (at 5% CO₂) containing Dulbecco's modified Eagle's medium supplemented with 10% fetal calf serum and 2 mM glutamine. For electrophysiological experiments, the cells were plated on a 35 x 10-mm culture dish containing 25-mm glass cover-slips. The cells were cultured in incubators (Galaxy, oxygen control model, RS Biotech, Irvine, UK). For the experiments H9C2 cells were infected with adenoviral constructs containing either green fluorescent protein (GFP, gift from C. Sunderland, University of Dundee; cells infected with GFP have served as control cells in this study), gly193-M-LDH (a catalytically inactive mutant of M-LDH, (Crawford et al., 2002a), Kir6.2, or Kir6.2AFA (a mutant form of Kir6.2 where the pore GFG was mutated into AFA leading to largely reduced K⁺ conductance, Van Bever et al., 2004). When intracellular and subsarcolemmal ATP levels were measured cells were infected with adenovirus containing luciferase and annexin VI-luciferase genes respectively. All these adenoviruses were generated and used as described in details in Jovanovic et al., 2009a, b. The recombinant SUR2A adenovirus (AV-SUR2A) was generated using the AdEasy XL Adenoviral Vector System (Stratagene). SUR2A gene was cloned into a shuttle vector pShuttle-CMV by PCR using the following primers containing restrict enzyme sites Bgl II / Xho l, sense, 5'- GCAGATCT GGC AGG CTG TTG GTA GCT CA-3', antisense, 5'-GCCTCGAG CTA CTT GTT GGT CAT CAC CA-3. The positive clones containing DNA inserts were linearized with Pme I and transformed into BJ5183-AD-1 competent cells to perform homologous recombination in Escherichia coli between the shuttle vectors carrying SUR2A gene and a large adenovirus-containing plasmid following electroporation. Recombinants were identified from single colonies, linearized, and then transfected into HEK293 cells to produce infective adenovirus virions. Adenoviral particles were obtained by cell extraction after 7-10 days of transfection, and the primary virus was further amplified by infection of AD-293 cultures, amplified virus stock is prepared by 4 rounds of freeze/thaw. The virus titer is determined using QuickTiter Adenovirus Titer Immunoassay Kit (Cell Biolabs, Inc) according to manufacturer's instructions. To infect H9C2 cells, a solution of recombinant adenovirus was mixed with culture medium, and cells were exposed to the virus with a multiplicity of 10 viral particles/cell for 48 h.

### Real time RT-PCR

Total RNA was extracted from heart of rat H9C2 cells using TRIZOL reagent (Invitrogen, Carlsbad, CA) according to the manufacturer's recommendations. Extracted RNA was further purified by RNeasy Plus Mini Kit (Qiagen, Crawley, UK) according to the manufacturer's instructions. Rat primers for all K_{ATP} channel subunits were designed as depicted on Table 1. All K_{ATP} channel subunits were measured using these primers apart on SUR2A that was measured by combining SUR2A rat (Table 1) and mouse (sense: ACTATGGAGTCCGAGAACTA, antisense: AGGTTTGGACCAGTATCACA) primers. The structural similarity between mouse and rat SUR2A is 93%, but the primers used for mouse SUR2A do not recognize RAT SUR2A and *vice verse.* Therefore, we have used mouse SUR2A to make adenoviral construct, so that we can examine the level of adenoviral SUR2A expression in host H9C2 cells. Top measure total SUR2A mRNA levels we have combined both sets of primers. The reverse transcription (RT) reaction was carried out with ImProm-II Reverse Transcriptase (Promega, Southampton, UK). A final volume of 20 µl of RT reaction containing 4 µl of 5× buffer, 3 mM MgCl₂, 20 U of RNasin® Ribonuclease inhibitor, 1 U of ImProm-II reverse transcriptase, 0.5 mM each of dATP, dCTP, dGTP, and dTTP, 0.5 µg of oligo(dT), and 1µg of RNA was incubated at 42°C for 1 h and then inactivated at 70°C for 15 min. The resulting cDNA was used as a template for real-time PCR. A SYBR Green I system was used for the RT-PCR and the 25 µl reaction mixture contained: 12.5 µl of iQ™ SYBR^{®} Green Supermix (2x), 7.5 nM each primers, 9 µl of ddH₂O, and 2 µl of cDNA. In principle, the thermal cycling conditions were as follows: an initial denaturation at 95°C for 3 min, followed by 40 cycles of 10 s of denaturing at 95°C, 15 s of annealing at 56°C, and 30 s of extension at 72°C. The real-time PCR was performed in the same wells of a 96-well plate in the iCycler iQ™ Multicolor Real-Time Detection System (Bio-Rad, Hercules, CA). Data was collected following each cycle and displayed graphically (iCycler iQ™ Real-time Detection System Software, version 3.0A, BioRad, Hercules, CA). Primers were tested for their ability to produce no signal in negative controls by dimer formation and then with regard to the efficiency of the PCR reaction. Efficiency is evaluated by the slope of the regression curve obtained with several dilutions of the cDNA template. Melting curve analysis tested the specificity of primers. Threshold cycle values, PCR efficiency (examined by serially diluting the template cDNA and performing PCR under these conditions) and PCR specificity (by constructing the melting curve) were determined by the same software. Each mouse cDNA sample was measured at three different quantities (and duplicated at each concentration, the corresponding no-RT mRNA sample was included as a negative control (blank). To determine relative mRNA expression (normalized to the wild type) we have used glyceraldehyde 3-phosphate dehydrogenase (GAPDH) as a control gene. The calculation of relative mRNA expression was performed as described (Pfaffl, 2001). The relative expression ratio(R) of SUR2A is calculated using equation R=(E_{K})^{ΔCP}ₖ^{(WT-TG)}/(E_{R})^{ΔCP}_{R}^{(WT-TG)} where E_{K} is the real time PCR efficiency of SUR2A gene transcript, E_{R} is the real time PCR efficiency of GAPDH gene, ΔC_{K} is the crossing point deviation of wild type-transgene (infected) of SUR2A gene transcript while ΔCP_{R} is the crossing point deviation of wild type-transgene of GAPDH gene transcript.

### Patch clamp electrophysiology

To monitor whole cell K⁺ current the giagaohm seal patch-clamp technique was applied in the perforated-patch whole cell configuration. H9C2 cells were superfused with Tyrode solution (in mM: 136.5 NaCl; 5.4 KCl; 1.8 CaCl₂; 0.53 MgCl₂; 5.5 glucose; 5.5 HEPES-NaOH; pH 7.4). All pipettes (resistance 3-5 MΩ), were filled with (in mM): KCl 140, MgCl₂ 1, HEPES-KOH 5 and and amphotericin B (Sigma, 240 µg/ml) (pH 7.3). For all cells monitored, the membrane potential was normally held at -40 mV and the currents evoked by a series of 400 ms depolarising and hyperpolarising current steps (-100 mV to +80mV in 20 mV steps) recorded directly to hard disk using an □Axopatch-200B amplifier, Digidata-1321 interface and pClamp8 software (Axon Instruments, Inc., Forster City, CA). The capacitance compensation was adjusted to null the additional whole-cell capacitative current. The slow capacitance component measured by this procedure was used as an approximation of the cell surface area and allowed normalisation of current amplitude (i.e. current density). Currents were low pass filtered at 2 kHz and sampled at 100 µs intervals.

### Cell survival assay

The survival of H9C2 cells were assayed using Multitox-Fluor Multiplex Cytotoxicity Assay (Promega). Briefly, H9C2 cells were plated in complete media (DMEM containing 10% FCS) in 96-well plate, the recombinant adenovirus (GFP as a control or other adenoviruses as stated in results section) was added to the wells at the multiplicity of infection of 10. After 48 hrs infection, the DNP was added to each well at the final concentration of 10 mM. To measure cell survival 6 hours later, the peptide substrate (GF-AFC) that can be cleaved only by live cells was added to the each well. Following 30 min-long incubation at 37°C, plates were measured using 1420 Multibabel Counter (Victor) plate reader, with excitation at 370 nm and emissions of 480 nm. The percentage of live cells was calculated based on the intensity of fluorescence according to the manufacturer instructions.

### Luciferase Assay

H9C2 cells were co-infected with adenoviruses containing genes encoding luciferase (to measure total ATP) or annexin-luciferase (to measure subsarcolemmal ATP) together with adenoviruses containing genes encoding K_{ATP} channel-forming proteins (as named in the Results section) 48 hours before luciferase assay. To measure luciferase luminesecence cells were mounted in 96-well plate in buffer with the following composition (in mM): 30 HEPES, 3 ATP, 15 MgSO₄, 10 DTT; pH: 7.4. Some of the cells were untreated while the others were treated with 10 mM DNP. The reaction for luciferase luminescence measurement was initiated by adding 100 µM of luciferin and the luminescence was measured on a plate reader 1420 Multibabel Counter (Victor). Luminescence was measured in the absence of DNP and after 1 hour of cell incubation with 10 mM DNP.

### Statistical analysis

Data are presented as mean±S.E.M, with n representing the number of analysed mice. Mean values were compared by the ANOVA followed by Student's *t*-test, Mann-Whitney rank sum test or by Chi-square test where appropriate using SigmaStat program (Jandel Scientific, Chicago, Illinois). P<0.05 was considered statistically significant.

### VIRAL VECTOR DESIGN

For transient expression of SUR2A we have generated SUR2A adenovirus.

The recombinant adenovirus was generated using the AdEasy XL Adenoviral Vector System (Stratagene). SUR2A gene was cloned into a shuttle vector pShuttle-CMV by PCR using the following primers containing restrict enzyme sites Bgl II / Xho I, sense, 5'-GCAGATCT GGC AGG CTG TTG GTA GCT CA-3', antisense, 5'-GCCTCGAG CTA CTT GTT GGT CAT CAC CA-3. PCR was performed by using the highest fidelity PfuUltra™ DNA polymerase (Stratagene) under the following condition: the PCR was run with a hot start for 2 min at 95°C, followed by 25 cycles of 0.5 min at 95°C, 0.5 min at 56°C, and 1 min at 72°C; and a final extension 10 min at 72°C. The PCR products were cloned between the Bgl II and Xho I sites of the pShuttle-CMV vector. The positive clones containing DNA inserts are linearized with Pme I, purified using agarose gel electrophoresis, and transformed into BJ5183-AD-1 competent cells to perform homologous recombination in Escherichia coli between the shuttle vectors carrying Sur2a gene and a large adenovirus-containing plasmid following electroporation. Recombinants are identified from single colonies with *Pac* I restriction enzyme (two DNA fragment, one is ∼30 kb, and the other is 4.5 kb). The positive recombinant plasmid is transformed into XL10-Gofd competent cells, purified using PureYield™ Plasmid Midiprep System (Promega), and linearized with *Pac* I. The linearized recombinant adenovirus plasmid is transfected into HEK293 cells to produce infective adenovirus virions using Lipofectamine 2000 (Invitrogen). Adenoviral particles were obtained by cell extraction after 7-10 days of transfection, and the primary virus was further amplified by infection of AD-293 cultures, amplified virus stock is prepared by 4 rounds of freeze/thaw. The virus titer is determined using QuickTiter Adenovirus Titer Immunoassay Kit (Cell Biolabs, Inc) according to manufacturer's instructions.

The process of generating SUR2A adenovirus is depicted in Figure 11.

For long term expression of we have used lentovirus. The commercial Trans-Lentiviral™ ORF Packaging System (Open Biosystems, Inc.) is used to generate SUR2A-Lentivirus. This kit allows creation of a replication-incompetent, HIV-1-based lentivirus which can be used to deliver and express SUR2A gene in either dividing or non-dividing mammalian cells. The Trans-Lentiviral ORF Packaging System possesses features which enhance its biosafety while allowing high-level gene expression in a wider range of cell types than traditional retroviral systems. The expression vectors contain a deletion in the 3' LTR (ΔU3) that does not affect generation of the viral genome in the producer cell line, but results in "self-inactivation" of the lentivirus after transduction of the target cell; The VSV-G gene from Vesicular Stomatitis Virus is used to pseudotype the vector particles, thus allowing production of a high titer lentiviral vector with a significantly broadened host cell range; Although the packaging plasmids allow expression *in trans* of genes required to produce viral progeny (e.g. *gag, pol, rev, tat, env*) in the TLA-HEK293T producer cell line, none of them contain LTRs or the packaging sequence. This means that none of the HIV-1 structural genes are actually present in the packaged viral genome, and thus, are never expressed in the transduced target cell. No new replication-competent virus has been shown to be produced. Figure 12 describes the general steps required to generate heart-specific Sur2A lentovirus using the Trans-Lentiviral™ ORF Packaging System.

Thus, a replication-incompetent lentivirus is produced by co-transfecting the Trans-Lentiviral packaging mix and the transfer vector pLEX-SUR2A into TLA-HEK293T cells; virus-containing supernatants after transfection is purified and concentrated to obtain a highter titer using Lentivirus Concentration and Purification Kit (Cell Biolabs, inc). Once the lentivirus enters the target cell, the viral RNA should be reverse-transcribed, actively imported into the nucleus, and stably integrated into the host genome.

### VECTOR ADMINISTRATION TO SUBJECTS/HEART

There are ranges of ways to introduce viral vectors into the heart. A direct injection into the heart muscle, in particular into the heart apex, secures a good and uniform expression of SUR2A. Viral vectors have been also delivered intravenously, intrarterial, intrapericardial and intramuscularly and the difference was in viral titers used. The amouns of viral particles used for injections varies and each individual animal can be tested for SUR2A expression and applied dose(s) corrected.

### COMPARATlVE NlCOTlNAMlDE ADMlNlSTRATlON TO SUBJECT/HEART

Nicotinamide was administered orally, i.e. as a dietary supplement. Mice were fed with nicotinamide-rich diet (ad libidum; 500 mg of nicotinamide per 1 kg of food) for 3 weeks. Estimated daily intake was 2.5 mg. For some in vitro experiments, cardiac cells were incubated for 24 hours in a DMEM medium containing 10% fetal calf serum, 2 m_{M} glutamine and 20 mM NAD or NADH.

All experiments have been done on male mice. Mice were on RM1 *ad libidum* diet (SDS, UK) and RM1 diet supplemented with nicotinamide (0.1, 0.5, 2.5, 12.5 g of nicotinamide per kg of diet which would lead to daily intake of 0.04 mg of nicotinamide in control diet and 0.4 mg, 2 mg, 10 mg and 50 mg in nicotinamide-rich diets). All mice were of similar age and weight. Mice were on these diets for a week. To assess physical endurance in these mice, we have used treadmill endurance test. A six lane treadmill (Columbus Instruments, Columbus, Ohio) was used to perform treadmill tests. The exercise-induced stress protocol consisted of a step-wise increase in velocity at constant incline. Before the experiments, animals were acclimatized to the treadmill for 30 minutes; 10 minutes at Om/min, 10 minutes at 3m/min followed by 10 minutes at 6m/min two days prior to the treadmill endurance test. The treadmill endurance test consisted of a step-wise increase in velocity over time at constant incline. The animal was made to run at 3, 5, 8 (all at m/min) for 3min intervals followed by 9, 10, 11, 12, 13, 14, 15 (all at m/min) for 5 min intervals and then at a constant velocity of 16m/min until exhaustion. After the test, mice were sacrificed, hearts harvested and SUR2A mRNA levels measured. More specifically, total RNA was extracted from cardiac ventricular tissue of transgenic and wild type mice using TRIZOL reagent (Invitrogen, Paisley, UK) according to the manufacturer's recommendations. Extracted RNA was further purified with RNeasy Mini Kit (Qiagen, Crawley, UK) according to the manufacturer's instruction. The for mouse SUR2A were designed as follows: sense, ACTATGGAGTCCGAGAACTA, antisense, AGGTTTGGACCAGTATCACA. The reverse transcription (RT) reaction was carried out with ImProm-II Reverse Transcriptase (Promega, Southampton, UK). A final volume of 20 µl of RT reaction containing 4 µl of 5× buffer, 3 mM MgCl₂, 20 U of RNasin® Ribonuclease inhibitor, 1 U of ImProm-II reverse transcriptase, 0.5 mM each of dATP, dCTP, dGTP, and dTTP, 0.5 5 µg of oligo(dT), and 1µg of RNA was incubated at 42°C for 1 h and then inactivated at 70°C for 15 min. The resulting cDNA was used as a template for real-time PCR. A SYBR Green I system was used for the RT-PCR and the 25 µl reaction mixture contained: 12.5 pi of iQ™ SYBR^{®} Green Supermix (2x), 7.5 nM each primers, 9 µl of ddH₂O, and 2 µl of cDNA. In principle, the thermal cycling conditions were as follows: an initial denaturation at 95°C for 3 min, followed by 40 cycles of 10 s of denaturing at 95°C, 15 s of annealing at 56°C, and 30 s of extension at 72°C. The real-time PCR was performed in the same wells of a 96-well plate in the iCycler iQ™ Multicolor Real-Time Detection System (Bio-Rad, Hercules, CA). Data was collected following each cycle and displayed graphically (iCycler iQ™ Real-time Detection System Software, version 3.0A, BioRad, Hercules, CA). Primers were tested for their ability to produce no signal in negative controls by dimer formation and then with regard to the efficiency of the PCR reaction. Efficiency is evaluated by the slope of the regression curve obtained with several dilutions of the cDNA template. Melting curve analysis tested the specificity of primers. Threshold cycle values, PCR efficiency (examined by serially diluting the template cDNA and performing PCR under these conditions) and PCR specificity (by constructing the melting curve) were determined by the same software. To determine relative SUR2A mRNA expression we have used glyceraldehyde 3-phosphate dehydrogenase (GAPDH) as a control gene; primers for GAPDH were: sense, 5'-GAGAATTCATGGTGAAGGTCGGTGTGAA-3', antisense, 5'-GCCTCGAGTTACTCCTTGGAGGCCATGT-3'. The relative expression ratio(R) of a gene encoding K_{ATP} channel subunit is calculated using equation R=(E_{K})^{ΔCP}ₖ^{(CD-ND)}/(E_{R})^{ΔCP}_{R}^{(CD-ND)} where E_{K} is the real time PCR efficiency of SUR2A gene transcript, E_{R} is the real time PCR efficiency of GAPDH gene, ACP_{K} is the crossing point deviation of control diet-nicotinamide diet of SUR2A gene transcript while ΔCP_{R} is the crossing point deviation of control diet-nicotinamide diet of GAPDH gene transcript.

### COMPARATIVE OESTROGEN ADMINISTRATION TO MALE SUBJECT/HEART

Cardiac cells were treated with 17β-estradiol (E2) applied in a concentration of 100 nM for 24 h. E2 can also be applied parenterally and for chronic administration it is particularly useful to implant E2 slow-release pellet (80 micrograms/kg/day).

### TREADMILL EXPERIMENTS

A six-track treadmill (Columbus Instruments, Columbus, OH) was used to study woild type and transgenic SUR2A mice. The exercise-stress protocol consisted of stepwise increases in either incline or velocity at 3-min intervals. A shock grid at the end of the treadmill delivered a mild painful stimulus to enforce running. Ten days before exercise stress, mice were acclimated daily for 45 min on a nonmoving treadmill followed by 15 min at a velocity of 3.5 m/min.

### EXAMPLE 1

### An increase in SUR2A mRNA levels in transgenic phenotype

We have separately analysed levels of SUR2A mRNA in male and female transgenic mice and littermate controls using real time RT-PCR. This method has revealed that level of SUR2A mRNA was significantly higher in hearts from female then male mice (the threshold cycle was recorded to be 23.38±0.84 and 24.33±0.20 for female and male mice respectively, n=6 for each, P=0.043, Fig. 1). In both genders, transgenic intervention has significantly increased mRNA levels of SUR2A (from 24.33±0.20 to 21.65±0.70, n=6, P<0.01 for males and from 23.38±0.84 to 20.03±0.0.26 for females, n=6, P<0.01, Fig. 1). The gender-specific difference in SUR2A mRNA levels has been retained in transgenic animals (P=0.038, n=6, Fig. 1). No difference was observed in mRNA levels of other K_{ATP} channel-forming subunits (Fig. 1).

### EXAMPLE 2

### Increased expression of SUR2A is cardioprotective in males, but not females

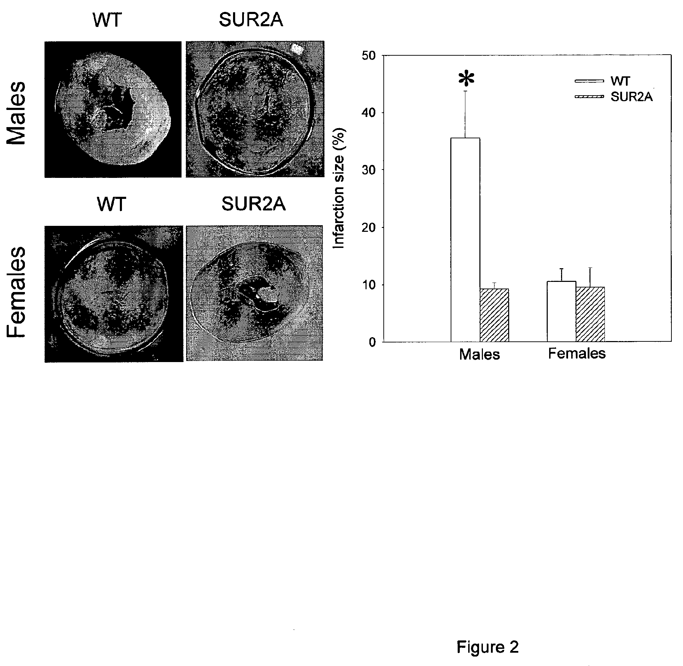

Ischaemia-reperfusion induced myocardial infarction in male wild type mice that was 35.5±8.2% of the area at risk zone (n=9, Fig. 2). The size of myocardial infarction was significantly smaller in male transgenic mice (10.5±2.2% of the area at risk zone, n=5, P=0.036, Fig. 2). On the other hand, hearts from female wild type mice were significantly more resistant to ischaemia/reperfusion then their male counterparts (the size of myocardial infarction was 9.2±1.1% of the area at risk zone, n=8, P=0.014 when compared to wild type males, Fig. 2). Increase in SUR2A expression did not further improve myocardial resistance to ischaemia/reperfusion (the size of myocardial infarction was 9.5±3.4% of the area at risk zone, n=6, P=0.806 when compared to wild type females, Fig. 2).

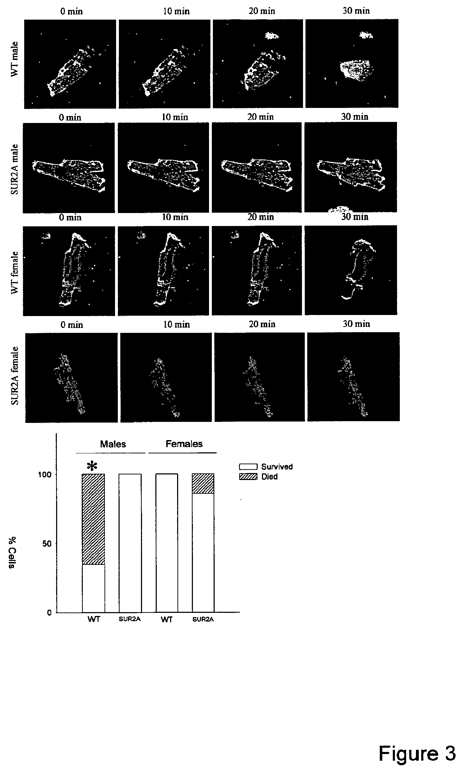

To examine whether improvement in myocardial resistance to ischaemia/reperfusion is due to the effect that SUR2A has on cardiomyocytes, we have tested the effect of hypoxia in cardiomyocytes from different gender and phenotype. When exposed to single cell hypoxia, 9 out of 14 cells from wild type males have died during first 30 min of hypoxia (Fig. 3). On the other hand, all 28 tested cells from male transgenic mice have survived 30 min-long hypoxia (Fig. 3). The difference between wild type and transgene males was statistically significant (P<0.001, Fig. 3). When cells from wild type females were analysed, all 14 examined cells have survived 30 min-long hypoxia. Increase in expression of SUR2A did not modify cellular response to hypoxia (12 out of 14 examined female transgenic cells have survived 30 min-long hypoxia, P=0.481 when compared to female wild type cells, Fig. 3).

### EXAMPLE 3

### SUR2A-mediated cardioprotection involve regulation of mitochondrial membrane potential

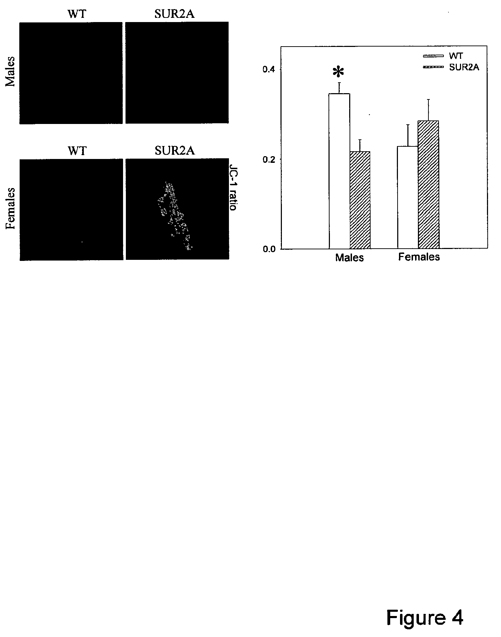

It has been recently suggested that a crucial event in cell death exposed to hypoxia/ischaemia is the opening of the mitochondrial permeability transition pore (mPTP; The mPTP is a non-specific megachannel in the inner mitochondrial membrane). The opening of mPTP is always associated with mitochondrial membrane depolarisation. It has been also shown that treatments inhibiting mitochondrial membrane depolarization protect the heart against iscahemia/reperfusion injury [7-9]. Here, we have measured mitochondrial membrane potential using JC-1, a well established dye used to measure this parameter. An increase in JC-1 green/red fluorescent ratio is indicative of mitochondrial membrane depolarisation. Under normoxic conditions, JC-1 ratio was 0.345±0.025 in cells from male wild type mice (n=6, Fig. 4A). Under similar conditions, JC-1 ratio was significantly lower in cells from male SUR2A mice (0.216±0.027, n=7, P=0.005, Fig. 4A). In female wild type cells JC-1 ratio was lower than those in male wild type cells (0.228±0.047, n=4, P=0.044 when compared to male wild type) and similar to JC-1 ratio in male SUR2A cells (P=0.816, n=4-7). Increased expression of SUR2A did not affect JC-1 ratio in female cardiomyocytes (JC-1 ratio was 0.284±0.047, n=5, P=0.430 when compared to female wild type).

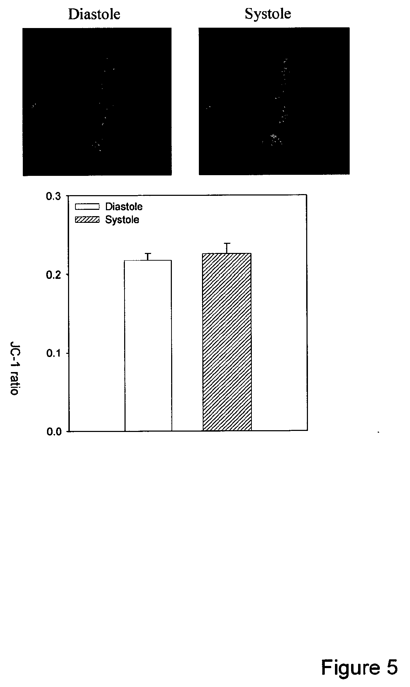

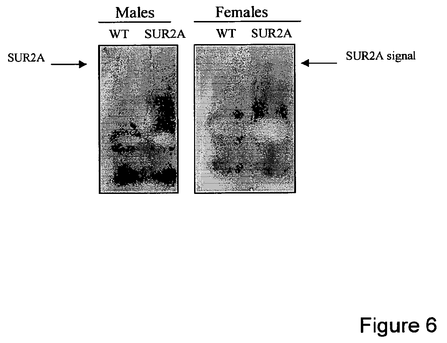

It has been previously shown that overexpression of SUR2A increases the number of sarcolemmal K_{ATP} channnels [14]. To test the possibility that changes in membrane potential might affects the mitochondrial membrane potential we have examined whether JC-1 ratio changes during systole and diastole, which is a processes associated with changes in membrane potential values. The average value of JC-1 ratio in diastole was 0.218±0.009 (n=12, Fig. 5) and 0.226±0.013 in systole (n=12, Fig. 5). This difference was not statistically significant (P=0.585). These results have suggested a possibility that increased expression of SUR2A might directly affect mitochiondrial membrane potential. If that would be the case, it was possible that mitochondria could express SUR2A. Therefore, we have immunoprecipitated and probed cardiac mitochondrial fraction with anti-SUR2 antibody. In mitochondrial fraction from male wild type mice there was apparent lack of signal that would correspond to SUR2A, while in any other phenotype this signal was present (Fig. 6).

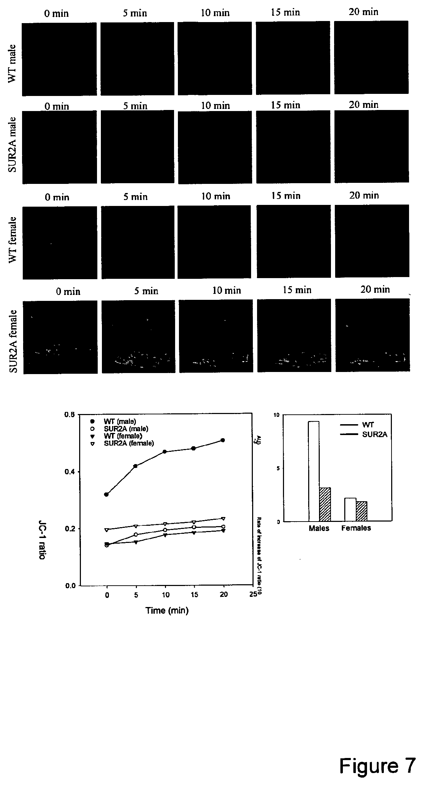

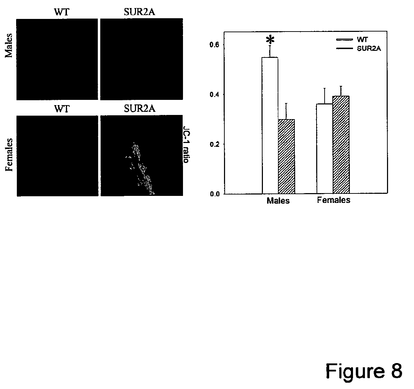

Furthermore, we have assessed the dynamics of changes of mitochondrial membrane potential during hypoxia. Typical examples of these experiments are depicted on Fig. 7. The rate of hypoxia-induced mitochondrial membrane depolarisation was significantly higher in cardiomyocytes from male wild type (Fig. 7) then in any other examined phenotype (Fig. 7). At the end of 30 min-long hypoxia JC-1 ratio in cells from wild type male mice was 0.547±0.048 (n=4, Fig. 8), which was significantly higher from those value obtained in male SUR2A mice (0.298±0.064, n=7, P=0.002, Fig. 8). On the other hand, JC-1 ratio in female cardiac cells after 30 min of hypoxia was 0.359±0.064 (n=4, Fig. 8), which was significantly lower then JC-1 ratio from their male counterparts (P=0.04). Increase of SUR2A in females did not significantly alter the value of JC-1 ratio at the end of hypoxia (0.390±0.041, n=4, P=0.699).

### COMPARATIVE EXAMPLE 4

### Nicotinamide-rich diet stimulates SUR2A expression in the heart

To determine whether nicotinamide-rich diet stimulates SUR2A expression in the heart, we have measured myocardial SUR2A mRNA levels in mice on control and nicotinamide-rich diet using real time RT-PCR as described [14]. This method has revealed that level of SUR2A mRNA was significantly higher in hearts from mice on nicotinamide-rich diet then in mice on control diet (the threshold cycle was recorded to be 29.71±0.88 and 23.57±1.47 for mice on control and nicotinamide-rich diet respectively, n=6 for each, P=0.004, Fig. 9).

Mice on control diet were able to stay on a treadmill for 67.3±6.2 min (n=6). Nicotinamide in doses 0.5, 2.5, 12.5 g/kg have increased the time on treadmill, but this was not statistically significant. The time on treadmill was 95.3±18.1 min (n=6, P=0.17 when compared with the control diet), 103.7±20.4 min (n=6, P=0.12 when compared with the control diet) and 88.2±12.5 min (n=6, P=0.17 when compared to the control diet) for .5, 2.5, 12.5 g/kg. In contrast, physical endurance of mice on 0.1 g/kg nicotinamide-rich diet was significantly increased to those in mice on control diet (the time on treadmill was 144.0±14.9 min, n=6, P=0.0007 when compared to the control diet). The levels of SUR2A mRNA in the heart were regulated by nicotinamide-rich diet and corresponded to physical endurance. Mice on control diet had the lowest level of SUR2A mRNA in the heart (the threshold cycle was 32.1±0.9, n=6) while mice on nicotinamide-rich diet on 0.1 g/kg had the highest myocardial SUR2A mRNA level (the threshold cycle was 21.8±0.8, n=6, P<0.001 when compared with control diet). Nicotinamide, when applied in a specific dose *per os,* increases physical endurance in mice. This effect on physical endurance was associated with up-regulation of SUR2A. This is illustrated in Figure 16.

### EXAMPLE 5

### Increased expression of SUR2A in the heart increases physical endurance

To test whether increase in SUR2A would improve physical endurance, we have assessed 4 transgenic SUR2A and 4 littermate wild type mice using treadmill exercise stress test, with stepwise escalating velocity and incline as described above. In wild type, the mean point of drop out (a moment when a mouse is physically incapable to sustain his position on a treadmill) was 24.6±2.3 min at speed 13.5 and incline 15. In transgenic mice, the mean point of drop out was 37.3±3.8 min at speed 20 and incline 20. Tolerated workload was calculated using formulae that takes into account weight of the animal, velocity and incline of treadmill as well as time that animal was able to sustain the exercise (see ref. 36). It was found that this parameter of physical endurance was almost 3-fold higher in transgenic mice compared to the wild type (1.47±0.14 vs 4.39±0.45 for wild type and transgenic respectively, n=4 for each, P<0.001).

### EXAMPLE 6

### Increased expression of SUR2A improves Ca²⁺ handling in heart cells

As increased physical endurance in mice overexpressing SUR2A in the heart has suggested a possibility that SUR2A-induced increase in cardiac capacity underlies this phenomenon. Therefore, we have measured intracellular levels of Ca²⁺ (a main parameter of intracellular metabolic condition in a heart cell; increase in intracellular Ca²⁺ is the main cause of heart impairement under any conditions, including , in cells exposed to isoprenaline (β-agonist used to mimick events happening during increased physical activity). When subjected to isoprenalione (100 µM), cardiomyocytes from wild type mice exhibited an increase in relative fluo-3 fluorescence reflecting an increase in calcium levels (127.2 ± 13.8, n=6) whereas, transgenic SUR2A mice exhibited a decrease in relative fluo-3 fluorescence (78 ± 16.6, n=6, p=0.04).

### EXAMPLE 7

### Infection with SUR2A increases the level of SUR2A in Heart H9C2 cells

We have analysed levels of SUR2A mRNA in control cells and cells infected with AV-SUR2A using real time RT-PCR. This method has revealed that infection with AV-SUR2A has significantly increased mRNA levels of SUR2A (cycling threshold was 23.20±0.10 in control cells and 21.05±0.05 in infected cells, n=4 for each, P<0.01, Fig. 13). No statistically significant difference was observed in mRNA levels of other K_{ATP} channel-forming subunits (Fig. 13). It is estimated that infected cells had 4.22 times more SUR2A mRNA then the control cells. Furthermore, we have probed anti-Kir6.2 immunoprecipitate of H9C2 extract with anti-SUR2 antibody. Using this strategy, we measured only those Kir6.2 and SUR2A subunits that physically assemble in sarcolemma to form a channel. Western blot has demonstrated that the level of myocardial SUR2A protein was increased in H9C2 cells in comparison to control cells (Fig. 13).

### EXAMPLE 8

### Infection with SUR2A increase cellular resistance to severe metabolic stress

DNP is known metabolic inhibitor that is used to induce metabolic stress in different cell types (Jovanovic et al., 1998). When control cells were treated with DNP (10 mM), only 43.3±1.8% (n=14) of cells have survived this insult (Fig. 14). Cells infected with SUR2A were significantly more resistant to DNP (10 mM) than control cells (58.9±2.0% of cells infected with SUR2A have survived DNP, n=8, P<0.001 when compared to the control, Fig. 14). On the other hand, infection with Kir6.2 alone did not have any effect on cellular survival under DNP (10 mM; 45.9%±2.6%, n=3, P=0.93 when compared to the control cells, Fig. 3) neither infection with SUR2A/Kir6.2 improved the effect of SUR2A alone (53.2%±4.1%, n=3, P=0.28 when compared with cells infected with SUR2A alone, Fig. 14).

### EXAMPLE 9

### Infection with SUR2A increases levels of subsarcolemmal ATP in H9C2 cells

Sarcolemmal K_{ATP} channel complex seem to contain, besides the channel subunits, glycolytic and other enzymes that catalyses reactions producing ATP. Anything that would increase the levels of ATP would improve cellular well being under different conditions of stress. Therefore, we have tested the levels of intracellular and subsarcolemmal ATP using luciferase and annexin-luciferase constructs respectively. Infection of SUR2A did not significantly change intracellular level of ATP (the intensity of luciferase luminescence was 144.8±5.3 AU in control and 142.8±8.9 AU in SUR2A cells, n=5, P=0.85, Fig. 15). However, infection with SUR2A did increase subsarcolemmal levels of ATP (the intensity of annexin-luciferase luminescence was 204.8±9.0 AU in control and 284.6±20.4 AU in SUR2A cells, n=5, P=0.01, Fig. 15). After treatment with DNP (10 mM) total intracellular ATP levels were not different in control cells and cells infected with SUR2A (the intensity of luciferase luminescence was 57.6±2.4 AU in control cells and 63.0±2.5 AU in SUR2A cells, n=5, P=0.16, Fig. 15). In contrast, subsarcolemmal ATP levels were significantly increased in SUR2A-infected cells (the intensity of annexin-luciferase luminescence was 71.8±2.1 AU in control cells and 96.8±2.4 AU in SUR2A cells, n=5, P<0.001, Fig. 15).

### References

1. Murry CE, Jennings RB, Reimer KA. Preconditioning with ischemia: a delay of lethal cell injury in ischemic myocardium. Circulation 1986; 74: 1124-36.
2. Yellon DM, Downey JM. Preconditioning of the myocardium: from cellular physiology to clinical cardiology. Physiol Rev. 2003; 83: 1113-51.
3. Murphy E, Steenbergen C. Gender-based differences in mechansims of protection in myocardial-reperfusion injury. Cardiovasc Reps. 2007; 75: 478-86.
4. Bennett SK, Redberg RF. Acute coronary sindromes in women: is treatment different? Should it be? Curr Cardiol Rep. 2004; 6: 243-52.
5. Weiss JN, Korge P, Honda HM, Ping P. Role of the mitochondrial permeability transition in myocardial disease. Circ Res. 2003; 93: 1385-94.
6. Halestrap AP, Clarke SJ, Javadov SA. Mitochondrial permeability transition pore opening during myocardial reperfusion-a target for cardioprotection. Cardiovasc Res. 2004; 61: 372-85.
7. Hausenloy DJ, Maddock HL, Baxter GF, Yellon DM. Inhibiting mitochondrial permeability transition pore opening: a new paradigm for myocardial preconditioning. Cardiovasc Res. 2002; 55: 534-43.
8. Javadov SA, Clarke S, Das M, Griffiths EJ, Lim KH, Halestrap AP. Ischaemic preconditioning inhibits opening of mitochondrial permeability transition pores in the perfused rat heart. J Physiol. (Lond) 2003; 549: 513-24.
9. Juhaszova M, Zorov DB, Kim SH, Pepe S, Fu Q, Fishbein KW, Ziman BD, Wang S, Ytrehus K, Antos CL, Olson EN, Sollott SJ. Glycogen synthase kinase-3beta□mediates convergence of protection signaling to inhibit the mitochondrial permeability transition pore. J Clin Invest. 2004; 113: 1535-49.
10. Burke MA, Mutharasan RK, Ardehali H. The sulfonylurea receptor, an atypical ATP-binding cassette protein, and its regulation of the KATP channel. Circ Res. 2008; 102: 164-76.
11. Ranki HJ, Budas GR, Crawford RM, Jovanovic A. Gender-specific difference in cardiac ATP-sensitive K+ channels. J Am Coll Cardiol 2001; 38: 906-15.
12. Ranki HJ, Budas GR, Crawford RM, Davies AM, Jovanovic A. 17β-estradiol regulates expression of KATP channels in heart-derived H9c2 cells. J Am Coll Cardiol. 2002; 40: 367-74.
13. Crawford RM, Jovanovic S, Budas GR, Davies AM, Lad H, Wenger RH, Robertson KA, Roy DJ, Ranki HJ, Jovanovic A. Chronic mild hypoxia protects heart-derived H9c2 cells against acute hypoxia/reoxygenation by regulating expression of the SUR2A subunit of the ATP-sensitive K+ channels. J Biol Chem. 2003; 278: 31444-55.
14. Du Q, Jovanovic S, Clelland A, Sukhodub A, Budas GR, Phelan K, Murray-Tait V, Malone L, Jovanovic A. Overexpression of SUR2A generates a cardiac phenotype resistant to ischaemia. FASEB J. 2006; 20: 1131-41.
15. Sukhodub A, Jovanovic S, Du Q, Budas GR, Clelland A, Shen M, Sakamoto K, Tian R, Jovanovic A. AMP-activated protein kinase mediates preconditioning in cardiomyocytes by regulating activity and trafficking of sarcolemmal ATP-sensitive K+ channels. J Cell Physiol. 2007; 210: 224-36.
16. Budas GR, Sukhodub A, Alessi DR, Jovanovic A. 3'phosphoinositide-dependent kinase-1 (PDK1) is essential for ischaemic preconditioning of the myocardium. FASEB J. 2006; 20: 2556-8.
17. Derecka K, Wang CK, Flint AP. Interactions between the cytomegalovirus promoter and the estrogen response element: implications for design of estrogen-responsive reporter plasmids. J Biomol Tench. 2006; 17: 218-27.
18. Ranki HJ, Crawford RM, Budas GR, Jovanovic A. Ageing is associated with decrease in number of sarcolemmal ATP-sensitive K+ channels in a gender-dependent manner. Mech Ageing Dev. 2002; 123: 695-705.
19. Kane GC, Liu XK, Yamada S, Olson TM, Terzic A. Cardiac KATP channels in health and disease. J Mol Cell Cardiol. 2005; 38: 937-43.
20. Budas GR, Jovanovic S, Crawford RM, Jovanovic A. 2004. Hypoxia-induced preconditioning in adult stimulated cardiomyocytes is mediated by the opening and traffcking of sarcolemmal KATP channels. FASEB J. 2004,18: 1046-8.
21. Carrasco AJ, Dzeja PP, Alekseev AE, Pucar D, Zingman LV, Abraham MR, Hodgson D, Bienengraeber- M, Puceat M, Janssen E, Wieringa B, Terzic A. Adenylate kinase phosphotransfer communicates cellular energetic signals to ATP-sensitive potassium channels. Proc Natl Acad Sci USA. 2001; 98: 7623-8.
22. Crawford RM, Ranki HJ, Botting CH, Budas GR, Jovanovic A. Creatine kinase is physically associated with the cardiac ATP-sensitive K+ channel in vivo. FASEB J 2002; 16: 102-4.
23. Crawford RM, Budas GR, Jovanovic S, Ranki HJ, Wilson TJ, Davies AM, Jovanovic A. M-LDH serves as a sarcolemmal KATP channel subunit essential for cell protection against ischemia. EMBO J. 2002; 21: 3936-48.
24. Jovanovic S, Du Q, Crawford RM, Budas GR, Stagljar I, Jovanovic A. 2005. Glyceraldehyde 3-phosphate dehydrogenase serves as an accessory protein of the cardiac sarcolemmal KATP channel. EMBO Rep. 2005; 6: 848-52.
25. Dhar-Chowdhury P, Maddison D H, Han SY, Jankowska D, Parachuru L, Morrissey A, Srivastava S, Liu W, Malester B, Yoshida H, Coetzee WA. The glycolytic enzymes, glyceraldehyde-3-phosphate dehydrogenase, triose-phosphate isomerase, and pyruvate kinase are components of the KATP channel macromolecular complex and regulate its function. J Biol Chem. 2005; 280: 38464-70.
26. Lembert N, Idahl LA, Ammon HP. KATP channel independent effects of pinacidil on ATP production in isolated cardiomyocytes or pancreatic beta-cell mitochondria. Biochem Pharmacol. 2003; 65: 1835-41.
27. Inoue I, Nagase H, Kishi K, Higuti T. ATP-sensitive K+ channel in the mitochondrial inner membrane. Nature. 1991; 352: 244-7.
28. Holmuhamedov E, Jovanovic S, Dzeja PP, Jovanovic A, Terzic A. Mitochondrial ATP-sensitive K+ channels modulate cardiac mitochondrial function. Am J Physiol Heart Circ Physiol. 1998; 275: H1567-76.
29. Sato T, Sasaki N, Seharaseyon J, O'Rourke B, Marban E. Selective pharmacological agents implicate mitochondrial but not sarcolemmal K(ATP) channels in ischemic cardioprotection. Circulation. 2000; 101: 2418-23.
30. Zhuo ML, Huang Y, Liu DP, Liang CC. KATP channel: relation with cell metabolism and role in cardiovascular system. Int J Biochem Cell Biol. 2005; 37: 751-64.
31. Marinovic J, Ljubkovic M, Stadnicka A, Bosnjak ZJ, Bienengraeber M. Role of sarcolemmal ATP-sensitive potassium channel in oxidative stress-induced apoptosis: mitochondrial connection. Am J Physiol Heart Circ Physiol. 2008; 294: H1317-25.
32. Zhou M, He HJ, Suzuki R, Liu KX, Tanaka O, Sekiguchi M, Itoh H, Kawahara K, Abe H. Localization of sulfonylurea receptor subunits, SUR2A and SUR2B, in rat heart. J Histochem Cytochem. 2007; 55: 795-804.
33. Halestrap AP, Clarke SJ, Javadov SA. Mitochondrial permeability transition pore opening during myocardial reperfusion-a target for cardioprotection. Cardiovasc Res. 2004; 61; 372-85.
34. Halestrap AP, Brennerb C. The adenine nucleotide translocase: a central component of the mitochondrial permeability transition pore and key player in cell death. Curr Med Chem. 2003; 10: 1507-25.
35. Andrienko T, Kuznetsov AV, Kaambre T, Usson Y, Orosco A, Appaix F, Tiivel T, Sikk P, Vendelin M, Margreiter R, Saks VA. Metabolic consequences of functional complexes of mitochondria, myofibrils and sarcoplasmic reticulum in muscle cells. J Exp Biol. 2003; 206: 2059-72.
36. Zingman LV, Hodgson DM, Bast PH, Kane GC, Perez-Terzic C, Gumina RJ, Pucar D, Bienengraeber M, Dzeja PP, Miki T, Seino S, Alekseev AE, Terzic A. Kir6.2 is required for adaptation to stress. Proc Natl Acad Sci USA 2002; 99: 13278-83.
37.

## Claims

1. A therapeutic agent for use in the treatment of myocardial ischaemia in a male subject that would benefit from enhanced expression or function of a sulfonylurea receptor polypeptide encoded by a nucleic acid molecule selected from the group consisting of:
i) a nucleic acid molecule comprising a nucleic acid sequence as represented in Figure 10a;
ii) a nucleic acid molecule that hybridizes to the nucleic acid sequence in (i) above under stringent hybridization conditions and which encodes a polypeptide with sulfonylurea receptor activity;
wherein said agent is:
an expression vector comprising said nucleic acid molecule encoding a sulfonylurea receptor polypeptide; wherein said nucleic acid molecule is operably linked to at least one promoter sequence, wherein the promoter is a hypoxia inducible promoter.

2. An agent for use according to claim 1 wherein said sulfonylurea receptor polypeptide comprises or consists of an amino acid sequence as represented in Figure 10b, or a variant amino acid sequence wherein said variant sequence varies from the amino acid sequence in Figure 10b by the addition deletion or substitution of at least one amino acid residue wherein said variant has sulfonylurea receptor activity.

3. An agent for use according to claim 1 wherein said vector is a recombinantly modified retroviridae or adenoviridiae.

4. An agent for use according to any preceding claim wherein said hypoxia inducible promoter is selected from the group consisting of: Adenosine A2B receptor (A2BR) promoter, Plasminogen activator receptor (uPAR) promoter, VEGF receptors (VEGFR1 and VEGFR2) promoter, Platelet-derived endothelial cell growth factor/thymidine phosphorylase (PDECGF/TP) promoter, nitric oxide synthase (NOS) promoter, Phosphoglycerate kinase-1 (PGK-1) promoter, Pyruvate kinase M (PK-M) promoter, Glucose transporter 1 (GLUT1) promoter, Hypoxia-inducible factor (HIF-1) promoter, Early growth response 1 (Egr-1) promoter, Nuclear factor kB (NFkB) promoter, Hepatocyte growth factor activator (HGFA) promoter, Vascular endothelial growth factor (VEGF) promoter, CXCL8 promoter, CCL11 promoter, Transforming growth factor-beta (TGF [beta]) promoter, procollagen promoter, Integrin-linked kinase (ILK) promoter, KIPDC1 promoter, Erythropoietin (EPO) promoter, Serine/Threonine Kinase-15 (STK15) promoter, the histone demethylase Jumonji domain containing 1A (JMJD1A) promoter, Endothelin-2 (EDN2) promoter, choline kinase (Chk) promoter, Sphingosine kinase 1 promoter, Carcinoembryonic antigen (CEA, ceacam[delta]) promoter, Monocyte chemoattractant protein-1 (MCP-1/CCL2) promoter, MCP-5 (CcH 2) promoter, prostate-specific antigen (PSA) promoter, c-Met promoter, Matrix metalloproteinases Class III beta-tubulin (TUBB3) promoter, Glutamine: fructose-6-phosphate amidotransferase (GFAT) promoter, Protein phosphatase I nuclear targeting subunit beta-secretase (BACE1) promoter and Plasminogen activator inhibitor-I (PAI-1) promoter.

5. An agent for use according to any preceding claim wherein SUR2A is provided with a mitochondrial targeting sequence that targets SUR2A to mitochondria, wherein said mitochondrial targeting sequence is preferably derived from a mitochondrial protein selected from the group consisting of:
anine/glyoxylate aminotransferase 1 (AGT), pyruvate dehydrogenase E1 alpha gene (PDH E1), manganese superoxide dismutase (MnSOD), Bax, mitochondrial protein kinase Cepsilon (PK Cepsilon), BfH, HCCS-1, translocase of outer membrane 22 (tom22), restorer gene (Rf-IA), trypanosoma cruzi (TcBPPI)1 a novel splice variant of the neuronal Bbeta regulatory subunit(Bbeta2), cAMP-dependent protein kinase anchoring protein (d-AKAP1), beta-naphthoflavone inducible cytochrome (P4501A1) p13II, a new human half-molecule ABC protein, designated M-ABC1 , vesicle-associated membrane protein-1 (VAMP-1), adenine nucleotide translocator (ANT), methionine sulfoxide reductase (MSRA), Human Factor B, Oxygen-regulated protein 150 (ORP150) Peroxiredoxin V (PRDX5), Protein tyrosine phosphatase interacting protein 51 (PTPIP51), cytochrome P4502B1, cytochrome P4501A1, The DNA helicase, Hmiip A-kinase-anchoring protein 1 (D-AKAP1) or Tim23p attached to a constitutive promoter and wherein the mitochondrial targeting sequence is preferably MSVLTPLLLRGLTGSARRLPVPRAKIHSL.

6. An agent according to any preceding claim that enhances the expression or activity of a sulfonylurea receptor for use in the treatment of a disease selected from the group consisting of: chronic obstructive pulmonary disease, heart failure, coronary artery spasm, dissecting aneurysms, infectious vasculitis, congenital defects in the coronary vascular system and pulmonary hypertension wherein the disease indirectly results in myocardial ischemia, and wherein the subject is male.

7. A method to diagnose a male subject suffering from or having a predisposition to myocardial ischemia comprising:
(i) determining the expression of SUR2A mRNA or protein in a blood or serum sample obtained from a male patient;
(ii) comparing the expression of SUR2A in said sample to a control sample wherein if the expression of SUR2A in said sample is lower than the expression of SUR2A in the control, the subject may benefit from administration of an agent according to any one of claims 1 to 5; and
(iii) determining whether said subject would benefit from administration of an agent according to any one of claims 1 to 5.

## Patentansprüche

1. Therapeutisches Mittel zur Verwendung bei der Behandlung von Myokardischämie bei einem männlichen Probanden, das von einer verstärkten Expression oder Funktion eines Sulfonylharnstoffrezeptor-Polypeptids, codiert durch ein Nukleinsäuremolekül profitieren würde, ausgewählt aus der Gruppe, bestehend aus Folgendem:
i) einem Nukleinsäuremolekül, umfassend eine wie in Figur 10a dargestellte Nukleinsäuresequenz;
ii) einem Nukleinsäuremolekül, das mit der Nukleinsäuresequenz in (i) oben unter strengen Hybridisierungsbedingungen hybridisiert, und das ein Polypeptid mit Sulfonylharnstoffrezeptor-Aktivität codiert;
wobei das Mittel Folgendes ist:
ein Expressionsvektor, umfassend das Nukleinsäuremolekül, das ein Sulfonylharnstoffrezeptor-Polypeptid codiert; wobei das Nukleinsäuremolekül funktionsfähig mit mindestens einer Promotorsequenz verknüpft ist, wobei der Promotor ein hypoxieinduzierbarer Promotor ist.

2. Mittel zur Verwendung nach Anspruch 1, wobei das Sulfonylharnstoffrezeptor-Polypeptid eine wie in Figur 10b dargestellte Aminosäuresequenz oder eine Aminosäuresequenzvariante umfasst oder aus dieser besteht, wobei sich die Sequenzvariante von der Aminosäuresequenz in Figur 10b durch zusätzliche Deletion oder Substitution von mindestens einem Aminosäurerest unterscheidet, wobei diese Variante Sulfonylharnstoffrezeptor-Aktivität aufweist.

3. Mittel zur Verwendung nach Anspruch 1, wobei der Vektor rekombinant modifizierte Retroviridae oder Adenoviridae ist.

4. Mittel zur Verwendung nach einem der vorhergehenden Ansprüche, wobei der hypoxieinduzierbare Promotor ausgewählt ist aus der Gruppe, bestehend aus: Adenosin-A2B-Rezeptor(A2BR)-Promotor, Plasminogen-Aktivator-Rezeptor(uPAR)-Promotor, VEGF-Rezeptoren(VEGFR1-und-VEGFR2)-Promotor, aus Blutplättchen stammendem Endothelzellwachstumsfaktor/Thymidinphosphorylase(PDECGF/TP)-Promotor, Stickoxidsynthase(NOS)-Promotor, Phosphoglyceratkinase-1(PGK-1)-Promotor, Pyruvatkinase-M(PK-M)-Promotor, Glukosetransporter-1(GLUT1)-Promotor, hypoxieinduzierbarem Faktor(HIF-1)-Promotor, Jugendwachstumsreaktion-1 (Egr-1)-Promotor, nuklearem Faktor-kB(NFkB)-Promotor, Hepatocytenwachstumsfaktor-Aktivator(HGFA)-Promotor, vaskulärem Endothelwachstumsfaktor(VEGF)-Promotor, CXCL8-Promotor, CCL11-Promotor, transformierendem Beta-Wachstumsfaktor(TGF-[beta])-Promotor, Prokollagen-Promotor, integrinverknüpftem Kinase(ILK)-Promotor, KIPDC1-Promotor, Erythropoietin(EPO)-Promotor, Serin/Threonin-Kinase-15(STK15)-Promotor, 1A enthaltendem Histon-Demethylase-Jumonji-Domäne(JMJD1A)-Promotor, Endothelin-2 (EDN2)-Promotor, Cholin-Kinase(Chk)-Promotor, Sphingosin-Kinase-1-Promotor, karzinoembryonalem Antigen(CEA, CEACAM [delta])-Promotor, weiße Blutkörperchen bindendem Monozyten-Protein-1(MCP-1/CCL2)-Promotor, MCP-5(CcH 2)-Promotor, prostataspezifischem Antigen(PSA)-Promotor, c-Met-Promotor, Matrixmetalloproteinase-Klasse-III-Beta-Tubulin(TUBB3)-Promotor, Glutamin: Fructose-6-Phosphat-Amidotransferase(GFAT)-Promotor, Protein-Phosphatase-I-Target-Untereinheit-Beta-Sekretase(BACE1)-Promotor und Plasminogenaktivatorinhibitor-I(PAI-1)-Promotor.

5. Mittel zur Verwendung nach einem der vorhergehenden Ansprüche, wobei SUR2A mit einer mitochondrialen Targeting-Sequenz bereitgestellt ist, die auf SUR2A-Mitochondrien zielt, wobei die mitochondriale Targeting-Sequenz vorzugsweise von einem mitochondrialen Protein stammt, das ausgewählt ist aus der Gruppe, bestehend aus: Anin/Glyoxylat-Aminotransferase-1(AGT), Pyruvatdehydrogenase-E1-Alpha-Gen (PDH E1), Mangan-Superoxiddismutase (MnSOD), Bax, mitochondrialm Proteinkinase-Cepsilon(PK-Cepsilon), BfH, HCCS-1, Translokase der äußeren Membran 22 (tom22), Wiederherstellungsgen (Rf-IA), Trypanosoma cruzi (TcBPPI) 1, einer neuartigen Spleißvariante der neuronalen regulatorischen Bbeta-Untereinheit (Bbeta2), cAMP-abhängige Proteinkinase-Ankerprotein (d-AKAP1), Beta-Naphthoflavon induzierbarem Cytochrom (P4501A1) p13II, einem neuen menschlichen Halbmolekül-ABC-Protein, bezeichnet als M-ABC1, vesikelassoziiertem Membranprotein-1 (VAMP-1),Adenin-Nukleotid-Translokator (ANT), Methionin-Sulfoxid-Reduktase (MRSA), Human-Faktor B, sauerstoffreguliertem Protein-150(ORP150)-Peroxiredoxin V (PRDX5), Protein-Tyrosin-Phosphatase interagierendem Protein 51 (PTPIP51), Cytochrom P4502B1, Cytochrom P4501A1, DNA-Helikase, Hmiip-A-Kinase-Ankerprotein 1 (D-AKAP1) oderTim23p, gebunden an einen konstitutiven Promotor, und wobei die mitochondriale Targeting-Sequenz vorzugsweise MSVLTPLLLRGLTGSARRLPVPRAKIHSL ist.

6. Mittel nach einem der vorhergehenden Ansprüche, das die Expression oder Aktivität eines Sulfonylharnstoff-Rezeptors zur Verwendung bei der Behandlung einer Erkrankung, ausgewählt aus der Gruppe, bestehend aus:
chronischer obstruktiver Lungenerkrankung, Herzinsuffizienz, Koronararterienspasmen, Dissektionsaneurysmen, infektiöser Vaskulitis, Geburtsfehler im Herzgefäßsystem und pulmonaler Hypertonie, verbessert, wobei die Erkrankung indirekt zu Myokardischämie führt, und wobei der Proband männlich ist.

7. Verfahren, um einen männlichen Probanden zu diagnostizieren, der an Myokardischämie leidet oder eine Prädisposition dafür aufweist, umfassend:
(i) Bestimmen der Expression von SUR2A-mRNA oder -Protein in einer Blut- oder Serumprobe, die von einem männlichen Probanden erhalten wird;
(ii) Vergleichen der Expression von SUR2A in der Probe mit einer Kontrollprobe, wobei, wenn die Expression von SUR2A in der Probe niedriger als die Expression von SUR2A in der Kontrolle ist, der Proband von der Verabreichung eines Mittels nach einem der Ansprüche 1 bis 5 profitieren kann; und
(iii) Bestimmen, ob der Proband von der Verabreichung eines Mittels nach einem der Ansprüche 1 bis 5 profitieren würde.

## Revendications

1. Un agent thérapeutique pour une utilisation dans le traitement de l'ischémie myocardique chez un sujet masculin qui bénéficierait d'une fonction ou d'une expression accrue d'un polypeptide récepteur de sulfonylurée codé par une molécule d'acide nucléique choisie dans le groupe consistant en :
i) une molécule d'acide nucléique comprenant une séquence d'acide nucléique tel que représenté sur la figure 10a ;
ii) une molécule d'acide nucléique qui s'hybride avec la séquence d'acide nucléique en (i) ci-dessus dans des conditions d'hybridation rigoureuses et qui code un polypeptide présentant une activité de récepteur de sulfonylurée ;
où ledit agent est :
un vecteur d'expression comprenant le fait pour ladite molécule d'acide nucléique de coder un polypeptide récepteur de sulfonylurée ; où ladite molécule d'acide nucléique est liée de manière opérante à au moins une séquence de promoteur, le promoteur étant un promoteur inductible par l'hypoxie.

2. Un agent pour une utilisation selon la revendication 1, où ledit polypeptide récepteur de sulfonylurée comprend ou consiste en une séquence d'acides aminés tel que représenté sur la figure 10b, ou une séquence d'acides aminés variante où ladite séquence variante varie de la séquence d'acides aminés de la figure 10b par l'ajout, la délétion ou la substitution d'au moins un résidu d'acide aminé, où ledit variant a une activité de récepteur de sulfonylurée.

3. Un agent pour une utilisation selon la revendication 1, où ledit vecteur consiste en des retroviridae ou des adenoviridiae modifiés par recombinaison.

4. Un agent pour une utilisation selon n'importe quelle revendication précédente où ledit promoteur inductible par l'hypoxie est choisi dans le groupe consistant en : promoteur du récepteur de l'adénosine A2B (A2BR), promoteur du récepteur activateur du plasminogène (uPAR), promoteur des récepteurs de VEGF (VEGFR1 et VEGFR2), promoteur de thymidine phosphorylase/facteur de croissance cellulaire endothélial dérivé des plaquettes (PDECGF/TP), promoteur de synthase d'oxyde nitrique (NOS), promoteur de phosphoglycérate kinase-1 (PGK-1), promoteur de pyruvate kinase M (PK-M), promoteur du transporteur de glucose 1 (GLUT1), promoteur du facteur inductible par l'hypoxie (HIF-1), promoteur de la réponse précoce au facteur de croissance 1 (Egr-1), promoteur du facteur nucléaire kB (NFkB), promoteur de l'activateur du facteur de croissance des hépatocytes (HGFA), promoteur du facteur de croissance endothélial vasculaire (VEGF), promoteur CXCL8, promoteur CCL11, promoteur du facteur de croissance transformant bêta (TGF [bêta]), promoteur de procollagène, promoteur de kinase liée à l'intégrine (ILK), promoteur KIPDC1, promoteur d'érythropoïétine (EPO), promoteur de sérine/thréonine kinase-15 (STK15), le promoteur de domaine histone déméthylase Jumonji contenant 1A (JMJD1A), promoteur d'endothéline 2 (EDN2), promoteur de choline kinase (Chk), promoteur de sphingosine kinase 1, promoteur d'antigène carcino-embryonnaire (CEA, ceacam[delta]), promoteur de protéine-1 chimiotactique des monocytes (MCP-1/CCL2), promoteur MCP-5 (CcH 2), promoteur d'antigène spécifique de la prostate (PSA), promoteur c-Met, promoteur de métalloprotéinases matricielles tubuline bêta de classe III (TUBB3), glutamine : promoteur de fructose-6-phosphate amidotransférase (GFAT), promoteur de sous-unité bêta-secrétase de ciblage nucléaire de protéine phosphatase I (BACE1) et promoteur de l'inhibiteur de l'activateur du plasminogène I (PAI-1).

5. Un agent pour une utilisation selon n'importe quelle revendication précédente où SUR2A est pourvu d'une séquence de ciblage mitochondrial qui cible SUR2A sur les mitochondries, ladite séquence de ciblage mitochondrial étant de préférence dérivée d'une protéine mitochondriale choisie dans le groupe consistant en : anine/glyoxylate aminotransférase 1 (AGT), gène alpha E1 de pyruvate déshydrogénase (PDH E1), superoxyde dismutase de manganèse (MnSOD), Bax, protéine kinase mitochondriale Cepsilon (PK Cepsilon), BfH, HCCS-1, translocase de membrane externe 22 (tom22), gène de restauration (Rf-IA), trypanosoma cruzi (TcBPPI1) un nouveau variant d'épissage de la sous-unité régulatrice Bbêta neuronale (Bbêta2), protéine d'ancrage de protéine kinase dépendante de cAMP (d-AKAP1), cytochrome inductible par naphthoflavone bêta (P4501A1) p13II, une nouvelle protéine ABC demi-molécule humaine, appelée M-ABC1, protéine membranaire associée à la vésicule-1 (VAMP-1), translocateur de nucléotides à adénine (ANT), méthionine sulfoxyde réductase (MSRA), facteur humain B, protéine régulée par oxygène 150 (ORP150), peroxyrédoxine V (PRDX5), protéine interagissant avec la protéine tyrosine phosphatase 51 (PTPIP51), cytochrome P4502B1, cytochrome P4501A1, l'ADN hélicase, protéine d'ancrage de kinase Hmiip 1 (D-AKAP1) ou Tim23p fixé à un promoteur constitutif et où la séquence de ciblage mitochondrial est de préférence MSVLTPLLLRGLTGSARRLPVPRAKIHSL.

6. Un agent selon n'importe quelle revendication précédente qui accroît l'expression ou l'activité d'un récepteur de sulfonylurée pour une utilisation dans le traitement d'une maladie choisie dans le groupe consistant en : broncho-pneumopathie chronique obstructive, insuffisance cardiaque, spasme coronarien, anévrismes disséquants, vasculite infectieuse, anomalies congénitales dans le système vasculaire coronaire et hypertension pulmonaire, où la maladie entraîne indirectement une ischémie myocardique, et où le sujet est masculin.

7. Un procédé pour diagnostiquer un sujet masculin souffrant ou présentant une prédisposition à des ischémies myocardiques comprenant :
i) déterminer l'expression de protéine ou d'ADNc SUR2A dans un échantillon sanguin ou sérique provenant d'un patient masculin ;
ii) comparer l'expression de SUR2A dans ledit échantillon avec un échantillon témoin où si l'expression de SUR2A dans ledit échantillon est inférieure à l'expression de SUR2A dans le témoin, l'administration d'un agent selon l'une quelconque des revendications 1 à 5 peut être bénéfique au sujet ; et
iii) déterminer si l'administration d'un agent selon l'une quelconque des revendications 1 à 5 serait bénéfique audit sujet.
